(19) Europäisches Patentamt
European Patent Office
Office européen des brevets

(11) EP 0 710 674 B1

(12) EUROPEAN PATENT SPECIFICATION

(45) Date of publication and mention of the grant of the patent:
**13.02.2002 Bulletin 2002/07**

(51) Int Cl.[7]: **C08B 37/16**, C12P 19/18

(21) Application number: **95250222.7**

(22) Date of filing: **13.09.1995**

# (54) Method for producing a glucan having cyclic structure

Verfahren zur Herstellung eines Glukans mit cyclischer Struktur

Méthode de préparation d'un glucane à structure cyclique

(84) Designated Contracting States:
**CH DE DK FR GB LI NL**

(30) Priority: **13.09.1994 JP 21855494**
**31.07.1995 JP 19564795**

(43) Date of publication of application:
**08.05.1996 Bulletin 1996/19**

(73) Proprietor: **Ezaki Glico Co., Ltd.**
**Osaka-shi, Osaka 555-8502 (JP)**

(72) Inventors:
- **Imanaka, Tadayuki**
  **Suita-shi, Osaka (JP)**
- **Terada, Yoshinobu**
  **Osaka (JP)**
- **Takaha, Takeshi**
  **Kobe-shi, Hyogo-ken (JP)**
- **Yanase, Michiyo**
  **Kako-gun, Hyogo-ken (JP)**
- **Okada, Shigetaka**
  **Ikoma-shi, Nara-ken (JP)**
- **Takata, Hiroki**
  **Suma-ku, Kobe-shi, Hyogo-ken (JP)**
- **Nakamura, Hiroyasu c/o Kowa-ryo**
  **Amagasaki-shi, Hyogo-ken (JP)**
- **Fujii, Kazutoshi c/o Kowa-ryo**
  **Amagasaki-shi, Hyogo-ken (JP)**

(74) Representative: **UEXKÜLL & STOLBERG**
**Patentanwälte Beselerstrasse 4**
**22607 Hamburg (DE)**

(56) References cited:
**EP-A- 0 675 137**

- **SZETLI J. 'Proceedings of the international symposium on cyclodextrins' 1982 , D. REIDEL PUBLISHING COMPANY , DORDRECHT, NL, BOSTON USA, LONDON GB K. HORIKOSHI et al.: "Industrial production of cyclodextrins", pages 25 - 39 * abstract ***

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 710 674 B1

Printed by Jouve, 75001 PARIS (FR)

## Description

## BACKGROUND OF THE INVENTION

1. Field of the Invention:

The present invention relates to the preparation of glucan or derivatives thereof useful as raw materials in the starch processing industry, in composition for drinks and foods, in composition for food additives, in composition for adhesion, or as starch substitutes for biodegradable plastic.
More specifically, the present invention relates to the preparation of glucan with a degree of polymerization of 50 or more having an inner branched cyclic structure portion and an outer branched structure portion and a method for producing the same.

2. Description of the Related Art:

Starch is a polymer substance used as a material for producing maltose, starch syrups, or cyclodextrin, a composition for foods and drinks, a composition for food additives, and a material for adhesion or biodegradable plastic. However, existing starch has various problems. For example, it is likely to retrograde and it has high viscosity. In general, starch has low solubility in water, and starch is therefore required to be heat treated or treated with an organic solvent, acid, or alkali so as to be dissolved in water.
Dissolved starch or gelatinized starch rapidly retrogrades to form an insoluble precipitate. When starch is retrograded, its physical properties such as viscoelasticity, adhesion change, water holding ability, shape holding properties, freezing resistance, and/or digestiveness in foods containing the retrograded starch decrease.
Furthermore, gelatinized starch has high viscosity. This is due to the fact that amylopectin in starch is composed of long chain molecules having a number of branches. In the case where maltose or cyclodextrin is produced using starch as a raw material, gelatinized starch is difficult to handle because of its high viscosity. For example, when gelatinized starch having a concentration at a certain level is transported through a pipe, the pipe becomes clogged with starch.
As described above, the characteristics (low solubility, retrogradation, and high viscosity) of existing starch limit the use thereof in foods and other fields.
Under these circumstances, a study was conducted for improving the solubility and retrogradation resistance of starch by allowing the starch to decompose into smaller molecules. As a result, retrogradation of starch was suppressed to a certain degree. However, it is difficult to prevent an excess decrease in molecular weight, and intrinsic characteristics of starch, which is originally a macromolecule, are lost. Furthermore, according to these methods, the reducing power of starch increases. When such starch is heated while being mixed with protein and amino acid so that when starch reacts, it turns colors. This problem also limits the use of starch.
On the other hand, a study for improving the solubility of starch without decomposing it into smaller molecules was conducted. Branching enzyme (Q-enzyme, EC 2.4.1.18) cleaves an α-1,4-bond of starch and allows an α-1,6-bond to be synthesized with the resulting starch by its transglycosylation reaction. Branching enzyme was allowed to react with starch to obtain water-soluble starch (Japanese Laid-Open Patent Publication No. 60-75295). However, the water-soluble starch obtained by this method is a polymer substance having a molecular weight, as high as starch used as a raw material, and therefore, cannot solve the problems mentioned above.
As a substitute for the above-mentioned starch, cyclic sugars composed of D-glucose, that is, cyclic glucan has been considered.
Cyclodextrins (CD) are known cyclic gulcans. Cyclodextrins are produced by allowing cyclodextrin glucanotrasferase (hereafter, referred to as CGTase) to react with starch or the like. When CGTase is allowed to react with starch, cyclodextrins (α-CD, β-CD, and γ-CD) having a degree of polymerization of 6 to 8, respectively, are usually produced. α-CD, β-CD, and γ-CD cannot, however, be substitutes for starch; thus, cyclodextrin having a higher degree of polymerization is desired.
Cyclodextrins having a degree of polymerization of 9 to 13 are synthesized by allowing CGTase to react with starch (Archives of Biochemistry and Biophysics, vol. 111, pp. 153-165 (1965)); however, their yield is extremely low.
Although Japanese Laid-Open Patent Publication No. 6-62883 discloses CD having a high degree of polymerization, its degree of polymerization is as high as 28. Furthermore, Archives of Biochemistry and Biophysics, vol. 111, pp. 153-165 (1965) describes that glucan (inner branched glucan or inner branched CD) having an α-1,6-bond in a cyclic structure composed of an α-1,4-glucoside bond, or glucan (outer branched glucan or outer branched CD) having an α-1,6-bond outside of a cyclic α-1,4-glucan is produced as a by-product of the action of CGTase on starch.
Japanese Laid-Open Patent Publication No. 6-62883 describes the structure of inner branched glucan by illustrating cyclodextrin having an inner branched structure. This Publication describes that inner branched cyclodextrin is present only in large cyclic cyclodextrin having degree of polymerization of 10 to 13 in which large strain is applied; and that the number of α-1,6-bonds present in the ring was one in accordance with analysis.

Japanese Laid-Open Patent Publication Nos. 63-46201 and 64-74997 describe the structure of an outer branched glucan and the method for producing the same. These Publications describe that branched dextrin can be produced by reacting debranching enzyme with the mixture of cyclodextrin and linear or branched dextrin. However, since cyclodextrin used for the reaction has no inner branched cyclic structure, glucan having an inner branched cyclic structure portion and an outer branched structure portion are not disclosed. Furthermore, the cyclic structure portion has a degree of polymerization of 6 to 8, and the outer branched structure portion is merely bound by maltooligosaccharide or branched maltooligosaccharide having a degree of polymerization of as high as 6.

As described above, although cyclodextrin may be considered a substitute for the above-mentioned starch, the CDs above cannot be a substitute for starch because of their small degree of polymerization. Although inner branched or outer branched CDs are disclosed, those branched CDs also have small degree of polymerization, and they are products of trace yields, so that they cannot be used as a substitute for starch.

In view of the above-mentioned circumstances, a method for easily producing cyclic glucan having a large molecular weight capable of being a substitute for starch is desired.

## SUMMARY OF THE INVENTION

The present invention provides a method for producing glucan, comprising the step of allowing a carbohydrate containing α-1,4-glucoside bonds and at least one α-1,6-glucoside bond to react with branching enzyme so as to generate glucan having a degree of polymerization of 50 or more, whose molecular weight is smaller than the molecular weight of the carbohydrate, and including an inner branched cyclic structure portion and an outer branched structure portion, wherein the inner branched cyclic structure portion is a cyclic structure portion formed from α-1,4-glucoside bonds and at least one α-1,6-glucoside bond and wherein the outer branched structure portion is a non-cyclic structure portion bound to the inner branched cyclic structure portion.

In one embodiment of the invention, the carbohydrate is starch.

In another embodiment of the invention, the carbohydrate is amylopectin.

In another embodiment of the invention, the degree of polymerization of the glucan is in the range of 50 to 5,000.

In another embodiment of the invention, a degree of polymerization of the inner branched cyclic structure portion of the glucan is in the range of 10 to 100.

In another embodiment of the invention, a degree of polymerization of the outer branched structure portion is 40 or more.

In another embodiment of the invention, a degree of polymerization of each unit chain of the outer branched structure portion of the glucan is in the range of 10 to 20 on average.

Alternatively a method for producing glucan is provided, comprising the step of allowing a carbohydrate having only α-1,4-glucoside bonds to react with branching enzyme so as to generate glucan having a degree of polymerization of 50 or more, whose molecular weight is smaller than the molecular weight of the carbohydrate, and including an inner branched cyclic structure portion and an outer branched structure portion, wherein the inner branched cyclic structure portion is a cyclic structure portion formed from α-1,4-glucoside bonds and at least one α-1,6-glucoside bond and wherein the outer branched structure portion is a non-cyclic structure portion bound to the inner branched cyclic structure portion.

According to another aspect of the invention, a composition for foods and drinks of the present invention includes the above-mentioned glucan.

According to another aspect of the invention, a composition for food additives of the present invention includes the above-mentioned glucan.

According to another aspect of the invention, an infusion composition of the present invention includes the above-mentioned glucan.

According to another aspect of the invention, a composition for adhesion of the present invention includes the above-mentioned glucan.

According to another aspect of the invention, an anti-retrogradation agent of the present invention includes the above-mentioned glucan.

Thus, the invention described herein makes possible the advantages of providing cyclic glucan, which is a material useful as a substitute for starch, having outstanding properties such as higher solubility in water, lower viscosity when being dissolved to form a solution, and not decomposing unlike ordinary starch; and a novel method for producing the cyclic glucan.

These and other advantages of the present invention will become apparent to those skilled in the art upon reading and understanding the following detailed description with reference to the accompanying figures.

## BRIEF DESCRIPTION OF THE DRAWINGS

Figure **1** is a schematic diagram illustrating an example of methods for producing glucan having an inner branched cyclic structure portion and an outer branched structure portion of the present invention.
Figure **2** shows gel filtration patterns of a reaction products obtained by the methods illustrated in Figure 1.
Figure **3** is a schematic diagram illustrating embodiment of glucans obtained by the method according to the present invention.
Figure **4** is schematic diagrams showing the conventionally considered reaction of CGTase: Figure **4A** shows the case where CGTase is allowed to act on amylose; and Figure **4B** shows the case where CGTase is allowed to act on amylopectin.
Figure **5** is a diagram showing the change in elution pattern of gel filtration chromatography of products when CGTase is allowed act on amylopectin.
Figure **6** is a diagram showing a novel action mechanism of CGTase.
Figure **7** is a graph showing that cyclic glucan other than CD is produced by the action of CGTase.
Figure **8** is a schematic diagram illustrating the decomposition of glucan by each enzyme, for determining the structure of glucan obtained by the method of the present invention.
Figure **9** shows amino acid sequences of Conserved Regions 1 and 4 among various amylases, and oligonucleotide primers for cloning branching enzyme corresponding to these sequences.
Figure **10** is a diagram showing a restriction enzyme map of λ-TBE102 containing branching enzyme gene.
Figure **11** is a diagram illustrating a method for constructing a plasmid containing branching enzyme gene.
Figure **12** is an elution pattern of glucan prepared in Example 1 on gel filtration column.
Figure **13** is a diagram showing the result of measurement of molecular weight of a glucoamylase-resistant component.
Figure **14** shows carbohydrate generated by the digestion of the glucoamylase-resistant component with various kinds of amylases.
Figure **15** is a diagram showing gel filtration chromatography of glucan obtained by using D-enzyme.
Figure **16** is a diagram showing gel filtration chromatography of glucan obtained by using CGTage.
Figure **17** is a diagram showing that glucan obtained in Example 1 has the effect of suppressing starch retrogradation.

## DESCRIPTION OF THE PREFERRED EMBODIMENTS

Hereinafter, the present invention will be described in detail.

(Outline of the present invention)

Figure **1** is a schematic diagram showing an example of methods for producing glucan with a degree of polymerization of 50 or more having an inner branched cyclic structure portion and an outer branched structure portion. In Figure **1**, a straight line in a horizontal direction and a curve represent α-1,4-glucan chains, and an arrow in a vertical direction represents α-1,6-glucoside bonds. The reference numeral **21** denotes a reducing end. A white triangle represents a glucoside bond attacked by enzyme, and a black triangle represents a glucoside bond newly synthesized by enzymatic action. In schematic diagrams described below, the straight line in the horizontal direction and the curve, the arrow in the vertical direction, the white triangle, and the black triangle respectively represent the same components as shown in Figure **1**.
The present invention is achieved based on the finding that when branching enzyme (BE), acts on amylopectin having α-1,4-glucoside bonds and α-1,6-glucoside bonds, glucan with a degree of polymerization of 50 or more having an inner branched cyclic structure portion and an outer branched structure portion denoted by the reference numerals **2, 5,** and **6** in Figure **1** are obtained. Figure **2** shows gel filtration patterns of the reaction products obtained in Figure 1. These reaction products have molecular weights which are much smaller than that of a material and larger than those of large cyclic CD represented by the reference numeral **3** of Figure **1**, an inner branched CD denoted by the reference numeral **8** of Figure **1**, an outer branched CD denoted by the reference numeral **9** of Figure **1**, a CD denoted by the reference numeral **4** of Figure **1**, and the like.
As denoted by the reference numerals **2, 5,** or **6** in Figure **1**, the glucan obtained by the method of the present invention has a degree of polymerization of 50 or more and is composed of an inner branched cyclic structure portion and an outer branched structure portion bound to the cyclic structure. Figure **3** shows embodiments of the present invention.
In the present specification, the inner branched cyclic structure portion refers to a cyclic structure portion formed from α-1,4-glucoside bonds and at least one α-1,6-glucoside bond.

In the present specification, the outer branched structure portion refers to a non-cyclic structure portion bound to the inner branched cyclic structure portion.

In the present invention, the above-mentioned term "glucan" includes glucan and derivatives thereof. Furthermore, according to the method of the present invention, the term "material" includes derivatized materials.

(Enzyme)

As the enzyme used in the present invention, 1,4-α-glucan branching enzyme (branching enzyme, Q-enzyme) capable of acting on carbohydrate having α-1,4-glucoside bonds and at least one α-1,6-glucoside bond to form glucan having a cyclic structure with a degree of polymerization of 50 or more can be used.

Considering the conventionally known action mechanism of this enzyme, it is surprising and unexpected that this enzyme produces glucan with a degree of polymerization of 50 or more having an inner branched cyclic structure portion and an outer branched structure portion.

Branching enzymes transfer a part of an α-1,4-glucan chain of starch-based carbohydrates to any C6 position of glucosyl residue of the glucan to form new α-1,6-linked branch. Thus, it was not expected that the branching enzyme would be capable of forming a cyclic structure.

(Branching enzyme)

Branching enzyme is present in various plants, animals and microorganisms such as bacteria. Its origin is not limited. Branching enzyme purified from E. coli having a plasmid encoding branching enzyme gene from thermophilic bacteria is preferred because of a high reaction optimum temperature, or branching enzyme derived from potato tuber is preferred because of facility in obtaining a great amount of enzyme.

The above-mentioned branching enzyme, can be used for producing the glucan of the present invention, even though it is a crude enzyme used in purification, if the enzymatic activity of endo-type amylases hydrolyzing an α-1,4- or α-1,6-glucoside bond in a starch molecule is not detected or is very weak.

Furthermore, the enzyme used in the present invention can be used for reaction even if it is immobilized, irrespective of whether it is a purified enzyme or a crude enzyme. The reaction can be performed by a batch method or a continuous method. As a method for immobilizing enzyme, methods known to those skilled in the art, such as a carrier bond method (e.g., a covalent bond method, an ion bond method, and a physical adsorption method), a cross-linking method or an including method (of a lattice type or of a microcapsule type), or the like can be used.

(Material to be used: substrate)

As a material used in the present invention, a carbohydrate having α-1,4-glucoside bonds and at least one α-1,6-glucoside bond can be used. Examples of the carbohydrate include starch, partially decomposed starch, amylopectin, glycogen, waxy starch, high-amylose starch, soluble starch, dextrin, starch hydrolysate, and enzymatically synthesized amylopectin with phosphorylase.

As the starch, any commercially available starch can be used. Examples of the starch include underground starch such as potato starch, sweet potato starch, arrowroot starch, and tapioca starch; and above-ground starch such as corn starch, wheat starch, and rice starch.

Examples of the partially decomposed starch include those obtained by partially hydrolyzing the above starch with enzyme or acid, and debranched starch.

As the amylopectin, waxy corn starch composed of 100% amylopectin is preferably used since the distribution of molecular weight of glucan to be produced becomes homogeneous. For example, amylopectin with a degree of polymerization of about 600 or more can be used as a material.

In the case of using branching enzyme, glucan having only α-1,4-bonds can also be used as a material. Examples of the glucan having only α-1,4-glucoside bonds include amylose, partially decomposed starch, debranched starch, enzymatically synthesized amylose with phosphorylase, and maltooligosaccharide. Amylose with a degree of polymerization of about 400 or more can be preferably used.

Furthermore, as a material, derivatives of the above-mentioned starch or partially decomposed starch can be used. Examples of the derivatives include those obtained by glycosylating, hydroxyalkylating, alkylating, acetylating, carboxymethylating, sulfurating, or phosphorylating at least one alcoholic hydroxyl group of the above-mentioned starch. Furthermore, mixtures of two or more kinds of these derivatives can also be used as a material.

(Enzymatic reaction on material)

Any steps of allowing the above-mentioned material to react with the above-mentioned enzyme can be used in the method for producing the glucan of the present invention, as long as the steps are conducted under conditions of pH, temperature, and the like allowing the glucan of the present invention to be generated. The concentration (substrate concentration) of the material can be determined considering the reaction conditions and the like.

In the case of using branching enzyme, the reaction pH is usually in the range of about 3 to about 11, preferably in the range of about 4 to about 10, and more preferably about 7 to about 9 in terms of reaction rate, efficiency, enzyme stability, and the like. The temperature is in the range of about 10°C to about 110°C, preferably in the range of about 20°C to about 90°C in terms of reaction rate, efficiency, enzyme stability, and the like. The substrate concentration is usually in the range of about 0.05% to about 60% by weight, preferably in the range of about 0.1% to about 30% by weight in terms of reaction speed, efficiency, handling of an enzyme solution, and the like. The amount of enzyme to be used is usually in the range of 50 to 10,000 units per gram of substrate.

A method for determining the unit of enzyme will be described in examples described later.

(Isolation and purification of product)

Various glucans having cyclic structures obtained in the above reaction can be separated by separation methods known to those skilled in the art such as chromatography (e.g., gel filtration chromatography, and HPLC), membrane filtration, and precipitation method using a solvent such as methanol and ethanol. One or more of the separating methods or purifying methods described above may be used.

According to the method of the present invention, the yield of the glucan from starch as a material is very high. In particular, when branching enzyme is used, the yield is almost 100%.

(Reaction product)

Glucan produced by the method of the present invention has a degree of polymerization of 50 or more and has an inner branched cyclic structure portion and an outer branched structure portion. The glucan has a degree of polymerization preferably in the range of about 50 to about 10,000, more preferably in the range of about 50 to about 7,000, and most preferably in the range of about 50 to about 5,000.

The degree of polymerization of the inner branched cyclic structure of the glucan produced by the method of the present invention is preferably in the range of about 10 to about 500, more preferably in the range of about 10 to about 100.

The inner branched cyclic structure portion of the glucan produced by the method of the present invention has at least one α-1,6-glucoside bond, usually 1 to about 200, and preferably about 1 to about 50.

The degree of polymerization of the glucan of this invention can be measured by gel filtration chromatography using amylose, whose degree of polymerization is known, as a standard. Furthermore, by using a differential refractometer and a low angle laser light scattering photometer, the degree of polymerization can be determined by the following principle. The output of the differential refractometer is proportional to the concentration of glucan, and the output of the low angle laser light scattering photometer is proportional to both of the degree of polymerization and the concentration of the glucan. Thus, by measuring the ratio of the outputs of both detectors, the degree of polymerization of glucan can be determined.

(Evidence that the glucan has a cyclic structure)

It can be confirmed by using glucoamylase whether or not the glucan obtained by the method of the present invention has an inner branched cyclic structure.

Exo-glucoamylase is an enzyme that successively hydrolyzes α-1,4-glucoside bonds from a non-reducing end of glucan such as starch. It is known that exo-glucoamylase is capable of hydrolyzing α-1,6-glucoside bonds from a non-reducing end at low speed. As shown in Figure **8**, amylose or amylopectin having no cyclic structure can be completely decomposed into glucoses **27** with exo-type glucoamylase. However, in glucans **25** and **23** having a cyclic structure in its molecule, only non-cyclic structure portion is decomposed, and their cyclic structure portion remains as material which is not decomposed with glucoamylase (hereinafter, referred to as glucoamylase-resistant component).

Whether or not this glucoamylase-resistant component (cyclic structure portion) has an inner branched structure can be determined by its sensitivity with respect to the debranching enzyme cleaving an α-1,6-bond. The cyclic glucan **26** having an inner branched cyclic structure (α-1,6-glucoside bond) can be completely decomposed into glucose **27** by combined use of debranching enzyme and glucoamylase.

On the other hand, the glucan **23** having a cyclic structure with no inner branched cyclic structure (i.e. having only α-1,4-glucoside bonds) cannot be decomposed with debranching enzyme and exo-type glucoamylase. The cyclic glucan **23** can be completely decomposed into glucose by combined use of endo-type α-amylase and glucoamylase.

By utilizing the above-mentioned characteristics, the inner branched cyclic structure portion of glucan, the outer branched (non-reducing) structure portion thereof, and the cyclic structure portion thereof having only α-1,4-glucoside bonds can be quantitated.

It can be confirmed by the following (1) to (6) characteristics that the glucan produced according to the present invention has an inner branched cyclic structure.

(1) The number of reducing ends does not increase, compared with a raw material (starch etc.). That is, a reducing end cannot be detected. The number of reducing ends can be determined by the modified Park-Johnson method of Hizukuri et al., Carbohydr. Res. 94:205-213 (1981).

(2) When glucoamylase is allowed to act on the glucan, a glucoamylase-resistant component remains. This component is not decomposed by glucoamylase, even after phosphatase treatment.

(3) The glucoamylase-resistant component is decomposed with isoamylase (produced by Hayashibara Biochemical Research Institute Co., Ltd., Okayama) hydrolyzing an α-1,6-glucoside bond in starch and becomes sensitive to the action of glucoamylase.

(4) The glucoamylase-resistant component is decomposed with endo-type α-amylase (produced by Nagase Seikagaku Kogyo Co., Ltd.), which hydrolyzes α-1,4-glucoside bonds in starch, and becomes sensitive to the action of glucoamylase.

(5) As a result of hydrolysis by endo-type α-amylase, isomaltosylmaltose (IMM) is generated. This agrees with the description that the minimum limit dextrin in the case where endo-type α-amylase is allowed to act on glucan having an α-1,6-glucoside bond is IMM (T. Yamamoto, Handbook of amylase and related enzymes, Pergamon Press, pp. 40-45 (1988)).

(6) The analysis of the molecular weight of the glucoamylase-resistant component by a laser ionization TOF-MS apparatus (manufactured by Shimazu Co., Ltd.) shows that the obtained value of the molecular weight coincides with the theoretical value of cyclic glucan and does not coincide with the theoretical value of non-cyclic glucan.

The detection of the glucoamylase-resistant component used for confirming the presence of an inner branched cyclic structure portion can be conducted as follows. For example, glucoamylase is added to the glucan generated by the above reaction, and the mixture thus obtained is allowed to react at about 40°C overnight. The reaction product is heated at 100°C for 10 minutes and an insoluble substance is removed by centrifugation. Then, 10-fold volume of ethanol is added to the supernatant and the polysaccharide remained in the supernatant is collected as a precipitate. This process is repeated again to obtain glucoamylase-resistant component. It is noted that a short period of time (e.g., 1 to 2 hours) is enough for the second glucoamylase treatment.

In the case where a raw material, such as starch, used in the present invention is partially modified with a phosphate group, pretreatment is required for the detection of the glucoamylase-resistant component. For example, a reaction product is dissolved in a 10 mM carbonate buffer (pH 9.4) containing 10 mM of $MgCl_2$ and 0.3 mM of $ZnCl_2$, and phosphatase is added to the resultant solution. Thereafter, 10-fold volume of ethanol is added to the reaction mixture and a precipitate is collected. A glucoamylase-resistant component can be obtained by applying the above-mentioned method to this precipitation.

The degree of polymerization and the structure of the glucoamylase-resistant component can be determined by analyzing carbohydrates generated by a reaction of glucoamylase-resistant component with glucan hydrolases, as described in the above-mentioned points (1) to (6). Examples of the hydrolases include glucoamylase, a combination of glucoamylase and isoamylase, and a combination of glucoamylase and α-amylase. The reaction is conducted as follows. The glucoamylase-resistant component is dissolved in distilled water so as to obtain a concentration of 0.2% (w/v), and the above-mentioned hydrolases were respectively added in an appropriate amount to the resultant solution. The solution is allowed to react at 30 to 45°C for an appropriate period of time (e.g., 1 hour). The decomposed glucoamylase-resistant component is subjected to a sugar analysis system (liquid transfer system: DX300, detector: PAD-2, column: Carbo Pac PA100, manufactured by Dionex) so as to be analyzed. The elution is conducted under the condition of, for example, a flow speed of 1 ml/minute, an NaOH concentration of 150 mM, a sodium acetate concentration of 0 minute-50 mM, 2 minutes-50 mM, 37 minutes-350 mM, 45 minutes-850 mM, and 47 minutes-850 mM. The degree of polymerization of the glucoamylase-resistant component and sugar generated by decomposition can be determined using this analysis.

(Regulation of degree of polymerization)

The degree of polymerization of glucan having an inner branched cyclic structure portion and an outer branched structure portion obtained by the method of the present invention can be regulated. For example, exo-type amylase, for example, glucoamylase is allowed to act on the obtained glucan so as to cleave a sugar chain of the outer branched structure portion, thereby obtaining glucan having a lower degree of polymerization. This method is also included in the present invention.

(Uses of glucan of the present invention)

The glucan of the present invention and the glucan obtained by the method of the present invention can be used for various uses of starch. In particular, they can be used as a composition for foods and drinks, a composition for food additives, a composition for an infusion, a composition for an adhesive, and an anti-retrogradation agent. In these uses, the glucan of the present invention can be used at a concentration appropriate for the respective uses.

Hereinafter, the present invention will be described by way of illustrative examples; however, the present invention is not limited by the examples.

Examples

A. Preparation of enzyme to be used and measurement of enzymatic activity

A-1: Preparation of branching enzyme: Preparation from potato and measurement of enzymatic activity

Potato tuber was homogenized in 20 mM Tris-HCl (pH 7.5) buffer containing 5 mM of 2-mercaptoethanol (buffer **A**) and centrifuged. The resulting solution was applied to Q-Sepharose column after being passed through a filter with a pore diameter of 0.45 µm. The column was washed with a buffer containing 150 mM of NaCl in buffer **A.** Then, branching enzyme was eluted with a buffer containing 450 mM of NaCl in buffer **A**. Then, ammonium sulfate was added to the elute so as to obtain a final concentration of 500 mM. The mixture was applied to Phenyl Toyopearl 650M (produced by Tosoh Corporation) column which was washed by a buffer containing 500 mM of ammonium sulphate in buffer **A**. By changing the concentration of ammonium sulphate in buffer **A** from 500 mM to 0 mM, elution was conducted. Fractions including branching enzyme were collected and dialyzed against buffer **A**. Dialyzate was applied to PL-SAX column (produced by Polymer Laboratory (U.K.)) equilibrated with buffer **A** and eluted by changing the concentration of NaCl in buffer **A** from 150 mM to 400 mM to collect fractions including branching enzyme.

The enzymatic activity was determined as follows. A 100 µl of a reaction solution containing 5 mM of Tris-HCl (pH 7.5), 0.05% (w/v) amylose, and enzyme was incubated at 30°C for 30 minutes, and the reaction was terminated by adding 2 ml of an iodine solution (containing 1 mg/ml of KI, 0.1 mg/ml of $I_2$, and 3.8 mM of HCl), and the absorbance of the solution at a wavelength of 660 nm was measured. The amount of enzyme decreasing the absorbance by 1% per minute is defined as 1 unit.

A-2: Preparation of branching enzyme-2: Preparation of recombinant branching enzyme from E. coli

Bacillus stearothermophilus TRBE 14 strain (Agency of Industrial Science and Technology, Biotechnology Industrial Research Institute; Accession No. FERM P-13916) was used to obtain a donor of branching enzyme gene.

Bacillus stearothermophilus TRBE 14 strain was cultured in 500 ml flask containing 100 ml of L-culture medium at 50°C overnight. After the completion of culture, the cells were collected by centrifugation. Chromosomal DNA was prepared from these collected cells by a phenol method (Saitoh and Miura, Biochimica et Biophysica Acta, 72, 619 (1963)). The chromosomal DNA thus obtained was partially degraded with restriction enzyme Sau3AI and subjected to NaCl density gradient centrifugation to collect DNA fragments of 10 kb or more. The collected DNA fragments of 10 kb or more were ligated with λ-EMBL3 vector (produced by Stratagene) previously cleaved with BamHI, using T4DNA ligase. A recombinant λ-phage suspension containing thus ligated DNA was prepared by using an in vitro package kit (Stratagene).

E. coli P2392 was incubated by shaker in an NZY-culture medium (1% NZ amine, 0.5% yeast extract, and 0.5% NaCl) supplemented with 10 mM of $MgCl_2$ at 37°C overnight. After incubation, cells were collected, suspended in 10 ml of 10 mM $MgCl_2$, and mixed with the recombinant λ-phage suspension. The mixture was kept warm at 37°C for 20 minutes. Then, the mixture was placed on an NZY agar culture medium and kept warm at 37°C overnight to obtain a plate with plaques.

A probe for selecting phage having a gene of interest from the recombinant λ-phage was prepared. In amino acid sequences of several kinds of branching enzymes, 4 conserved regions (Conserved Regions 1, 2, 3, and 4) which have already been found in α-amylase or the like are known to be present (J. Biol. Chem., 267, 18447 (1992)). Two kinds of DNA primers, a sequence (5' GAYTGGGTNCCNGSNCAYTTY 3': SEQ ID NO: 1) corresponding to a sequence of Conserved Region 1 shown in Figure **9** and a sequence (3' WSNGTRCTRCTYCANCANGTR 5': SEQ ID NO: 2) corresponding to a sequence in the vicinity of Conserved Region 4 were synthesized. These synthetic primers were allowed to react with the chromosomal DNA of 10 kb or more obtained in the above, using Tth DNA polymerase (produced by Toyobo Co., Ltd.), whereby a DNA fragment sandwiched by the two synthetic primers was amplified. The reaction was repeated for 30 cycles, each cycle including 94°C for 1 minute; 45°C for 1 minute; and 72°C for 1 minute. The amplified DNA fragment was about 560 bp. The amplified DNA fragment was ligated with pUC19 treated with restriction enzyme SmaI to obtain recombinant plasmid pTBE3. A radio-labelled probe for isolating branching enzyme gene was prepared from the recombinant plasmid pTBE3 by using a DNA labelling kit for multiprime method (Pharmacia).

A nylon filter (Amersham Corp.) was attached to the plate with plaques of the recombinant λ-phage. The plate was alkali-treated to denature the recombinant λ-phage DNA in the plaques, whereby the DNA was immobilized on the filter. The resultant filter was soaked in the solution of radio-labelled probe prepared in the above and hybridization was done at 65°C for 16 hours. The filter was thoroughly washed, dried, and attached to an X-ray film so as to select recombinant λ-phage containing branching enzyme genes. The recombinant λ-phage thus obtained was named λ-TBE102. Figure **10** shows a restriction enzyme map of λ-TBE102.

A plasmid containing branching enzyme gene was produced by a method shown in Figure **11**. First, a DNA fraction was recovered from λ-TBE102, cleaved with SalI, and subjected to agarose gel electrophoresis. The DNA was transferred to a nitrocellulose filter by a conventional method and hybridized at 65°C for 16 hours, using the above-mentioned probe. A 4 kb SalI fragment was hybridized. The 4 kb SalI fragment was isolated and introduced into an SalI site of pBR322 to produce pTBE31.

Next, 1.4 kb of a SalI-EcoRI fragment was isolated from pTBE31 and was ligated to plasmid pUC118 treated with SalI-EcoRI to prepare pUC118SE. pUC118SE was cleaved with EcoRI and cleaved sites were made blunt ends with T4DNA polymerase. Hind III linker was introduced into the cleaved sites to form pUC118SEH.

On the other hand, about 5 kb of a XhoI DNA fragment containing the above 4 kb SalI fragment was introduced into SalI site of pBR322 to form pTBE51. A SalI-Aor51HI DNA fragment was cut out from pTEB51 and ligated to pUC118 cleaved with SmaI-SalI to form pUC118AS.

About 1 kb of BamHI-SalI DNA fragment was isolated from pUC118AS and ligated to pUC118SEH treated with BamHI-SalI to form pTBE821. This plasmid pTBE821 encoded an amino acid sequence (SEQ ID NO: 3) of branching enzyme.

This plasmid pTBE821 was introduced into E. coli TG-1 by a conventional method. E. coli TG-1 was cultured and centrifuged at 8,000 rpm for 5 minutes to obtain cells. These cells were suspended in 50 mM of Tris-HCl (pH 7.0) containing 5 mM of 2-mercaptoethanol (buffer **B**) and disrupted with sonication. Undissolved substance was removed by centrifugation at 12,000 for 10 minutes, the suspension was heated at 60°C for 15 minutes and precipitates such as denatured protein was removed by centrifugation at 12,000 rpm for 10 minutes. The supernatant thus obtained was dialyzed against buffer **B** and then applied to Q-Sepharose column (manufactured by Pharmacia). The column was washed with a buffer containing 100 mM of NaCl in buffer **B**. Branching enzyme was eluted with a buffer containing 200 mM of NaCl in buffer **B.** Then, ammonium sulfate was added to the eluate so as to obtain a final concentration of 170 mM. The mixture was applied to Phenyl Toyopearl 650M (Tosoh Corporation) column and branching enzyme was eluted with buffer **B** to obtain branching enzyme.

The measurement of the enzymatic activity was conducted in the same way as in the case of potato branching enzyme, except that the reaction temperature was 50°C.

A-3: Preparation of D-enzyme and measurement of enzymatic activity (comparative Example)

D-enzyme was purified by the method described in Takaha et al., J. Biol. Chem., vol. 268, pp. 1391-1396 (1993). First, potato tuber was homogenized in buffer **A** and centrifuged. The resultant solution was applied to a Q-Sepharose column (16 x 100 mm Pharmacia) after being passed through a membrane having pores of 0.4 μm. The column was washed with the buffer containing 150 mM of NaCl in buffer **A.** D-enzyme was eluted with a buffer containing 450 mM of NaCl in buffer **A.** After elution, the eluate was dialyzed against buffer **A** and ammonium sulfate was added to the dialyzate so as to obtain a final concentration of 500 mM. This solution was loaded onto a Phenyl Toyopearl 650M (Tosoh Corporation) column (10 x 100 mm), and the elution was conducted by changing the concentration of ammonium sulfate in buffer **A** from 500 mM to 0 mM. Active fractions of D-enzyme were collected and dialyzed against buffer **A**. Dialysate was concentrated by an Amicon Centricon 30 microconcentrator, and the concentrated dialysate was applied to PL-SAX HPLC column (Polymer Laboratory U.K.) and eluted in a 150 to 400 mM of NaCl linear concentration gradient in buffer **A**. Active fractions were collected from the eluate thus obtained and concentrated by the above-mentioned Amicon Centricon 30 microconcentrator.

The measurement of the enzymatic activity was determined by a method using glucose oxidase (Barham et al., (1972) Analyst 97:142). According to this method, 100 μl of a reaction mixture containing 100 mM of Tris-HCl (pH 7.0), 5 mM of 2-mercaptoethanol, 1% (W/V) of maltotriose, and enzyme was allowed to react at 37°C for 10 minutes. The reaction solution was heated for 3 minutes in boiling water to stop the reaction. Glucose liberated by the reaction was measured. The amount of enzyme used for generating 1 μmol of glucose per minute is defined as 1 unit.

A-4: Preparation of CGTase (comparative Example)

CGTase derived from Alkalophilic Bacillus sp. A2-5a (hereinafter, referred to as A2-5a strain) was used. Japanese Laid-Open Patent Publication No. 7-107972 discloses the properties of a CGTase producer Alkalophilic Bacillus sp. A2-5a, which was deposited by the Applicant in the Agency of Industrial Science and Technology, Biotechnology Industrial Research Institute and assigned accession No. FERM P-13864.

A method for purifying CGTase derived from an A2-5a strain was as follows:

The A2-5a strain was cultured in an AL liquid culture medium (1% soluble starch, 4% corn steep liquor, 0.1% $K_2HPO_4$, 0.02% $MgSO_4$, $7H_2O$, 1% $Na_2CO_3$, pH=10.0) at 33°C for 24 hours. After incubation, culture broth was centrifuged to remove cells therefrom and the culture supernatant thus obtained was collected. Twenty grams of starch was added to 1.6 L of the culture supernatant, the mixture was stirred at 4°C for 16 hours, and CGTase was allowed to adsorb to starch granule. CGTase absorbed to starch particles was applied to a column, and the column was washed 5 times with 100 ml of 22.8% ammonium sulfate solution. Thereafter, CGTase was eluted 5 times with 100 ml of 33 mM $Na_2HPO_4$. Ammonium sulfate was added to the eluate so as to obtain a final concentration of 57%. The precipitate thus obtained was collected and dialyzed against 20 mM of Tris-hydrochloride buffer (pH 7.5). The entire solution thus obtained was loaded onto a Q-Sepharose column (8 ml) pre-equilibrated with 20 mM of Tris-hydrochloride buffer (pH 7.5), and the column was washed with 50 ml of Tris-hydrochloride buffer containing 0.4 M of NaCl. By changing the concentration of NaCl in the buffer from 0.4 M to 1 M, CGTase was eluted. Then, active fractions of CGTase were collected to obtain purified CGTase derived from the A2-5a strain.

A-5: Preparation of immobilized CGTase (comparative Example)

One g of carrier Quitopal BCW-3503 (Fuji Spinning Co., Ltd.), washed with distilled water, for immobilization of enzyme and 5 ml of 20 mM of sodium acetate buffer (pH 5.5) containing 100 units of CGTase was incubated with gentle stirring at room temperature for 2 hours, whereby CGTase was adsorbed to the carrier. The suspension was filtrated and the CGTase activity of the filtrate was measured. The CGTase activity was hardly detected. Therefore, it was considered that most of the CGTase was bound to the carrier. This CGTase-bound carrier was used for reaction.

The CGTase activity was measured as follows: 1.5% soluble starch solution (adjusted to pH 5.5 with 20 mM of sodium acetate buffer) was placed in a constant temperature bath previously set at 40°C. Then, CGTase was added to this starch solution. After 10 minute, 0.5 ml of solution containing 0.5 N acetic acid-0.5N HCl (5:1 v/v) was added to the reaction solution (0.25 ml) to stop the reaction. Then, 0.1 ml of the solution was taken and a solution containing 0.005% $I_2$ and 0.05% KI was added thereto. The resultant solution was stirred and allowed to stand at room temperature for 20 minutes. The absorbance of this solution at 660 nm was measured. At this time, a solution with no CGTase added was prepared as a blank, and the same procedure was conducted. Under this condition, the amount of enzyme causing the decrease in absorbance by 10% per minute at 660 nm was determined to be one unit.

B. Production of glucan using branching enzyme

(Example 1: Preparation of glucan)

Five grams of commercially available waxy corn starch (having an average degree of polymerization of 30,000 or more) was suspended in 40 ml of 5 mM sodium phosphate buffer (pH 7.5), and the suspension was gelatinized in a hot bath at 100°C, followed by being cooled to about 50°C. Then, 2000 units of purified, recombinant branching enzyme were added to this gelatinized solution and allowed to react 50°C for 6 hours.

The reaction solution was heated at 100°C for 20 minutes, and denatured enzyme protein was removed therefrom by centrifugation at 10,000 rpm for 15 minutes. Twice volume of ethanol was added to the supernatant, and the mixture was allowed to precipitate. The precipitate was lyophilized to obtain about 4.8 g of powdery glucan.

(Example 2: Measurement of degree of polymerization)

The degree of polymerization was measured by gel chromatography, using a differential refractometer and a low angle laser light scattering photometer. The glucan obtained in the above reaction was dissolved so as to be 0.5% (W/V) in 0.1 M phosphate buffer (pH 6.2) containing 1.5% acetonitrile and 0.02% sodium azide. Then, 200 µl of this solution was applied to a column obtained by connecting Asahipak GS520, Asahipak GS320 (both manufactured by Asahi Chemical Industry Co., Ltd.), and TSK-G2000PW (Tosoh Corporation).

Figure **12** shows an elution pattern. As shown in Figure **12**, the degree of polymerization of glucan thus produced was in the range of about 50 to 5,000, and peaks **S** and **L** were found at 460 and 770, respectively. The weight average degree of polymerization was 900.

(Example 3: Determination of structure of glucan obtained in Example 1)

3-1: Quantitation of reducing end

The quantitation of reducing end of glucan obtained in Example 1 was conducted by the modified Park-Johnson method of Hizukuri et al. Compared with the waxy corn starch as a raw material, the increase in the number of reducing ends was not detected. Since the degradation of the material into lower molecules without increasing reducing ends can only be explained by the cyclizing reaction, it is concluded that the glucan has one cyclic structure in its molecule.

3-2: Obtaining glucoamylase-resistant component

The detection of glucoamylase-resistant component was conducted as follows:

First, 500 mg of the glucan obtained in Example 1 was thoroughly dissolved in 500 µl of DMSO. To this glucan solution, 500 µl of 1 M sodium acetate buffer (pH 5.5), 3 ml of distilled water, and 1 ml of 50 units/ml glucoamylase were added, and the mixture was reacted at 40°C for 3 hours. The reaction mixture was heated at 100°C for 10 minutes, and undissolved substance was removed therefrom by centrifugation at 10,000 rpm for 15 minutes. Then, 10-fold volume of ethanol was added to the supernatant, and the mixture was centrifuged at 10,000 rpm for 15 minutes to collect a precipitate. The precipitate thus obtained was dried and dissolved in 1 ml of distilled water. Fifty units of glucoamylase were added to the solution thus obtained, followed by being reacted at 40°C for 3 hours. The reaction solution was heated at 100°C for 10 minutes, and undissolved substance was removed by centrifugation at 10,000 rpm for 15 minutes. Then, 10-fold volume of ethanol was added to the supernatant, and the mixture was centrifuged to collect a precipitate. The precipitate thus obtained was dried to obtain 13 mg of powdery glucoamylase-resistant component. This suggested that the glucan obtained in Example 1 had a cyclic structure.

3-3: Measurement of degree of polymerization of glucoamylase-resistant component

The glucoamylase-resistant component thus obtained was subjected to gel filtration chromatography using a differential refractometer and a low angle laser light scattering photometer to obtain an average degree of polymerization of 49. This value was smaller than the average degree of polymerization of 900 of the glucan before being decomposed, indicating that an outer branched structure portion degradable with glucoamylase was present. From this glucoamylase-resistant component, low molecule portions were obtained by gel filtration chromatography. The low molecule portions were subjected to laser ionization TOF-MS apparatus (Kompact Maldi, manufactured by Shimadzu Corporation). As shown in Figure 13, many peaks were observed. The molecular weight of these portions was well matched with the theoretical value of glucans having cyclic structures, each having a degree of polymerization different from each other by one. An error was within the limits (±0.2%) of the apparatus; the error was not matched with the theoretical value of glucan not having a cyclic structure. In Figure **13**, the molecular weight at the lowest peak is 2455, which is matched with the molecular weight of cyclic glucan with a degree of polymerization of 15 with a sodium ion added. In addition, a peak at which the molecular weight was matched with that of cyclic glucan with a degree of polymerization of 16 to 36 was detected.

3-4: Digestion of glucoamylase-resistant component with various kinds of amylases

The glucoamylase-resistant component obtained in the above was dissolved in distilled water so as to be 0.4% (w/v). Then, amylases shown in Figure **14** were respectively added to 50 µl of the solution so as to be 60 µl with a concentration shown in Figure **14**. The resulting solutions were allowed to react at 40°C overnight. The reaction solutions were heated at 100°C for 5 minutes, and denatured protein was removed by centrifugation at 12,000 for 5 minutes. Each of the reaction products was subjected to a carbohydrate analysis system of Dionex. The conditions described previously were used.

As shown in Figure **14**, the glucoamylase-resistant component was not decomposed with single use of glucoamylase; however, it was decomposed with α-amylase (Nagase Biochemical Industrial Co., Ltd.), i.e., endo-type amylase hydrolyzing α-1,4-glucoside bonds in a starch molecule, and IMM was detected. The glucoamylase-resistant component was also decomposed by the combined use of glucoamylase and isoamylase (Hayashibara Biochemical Research Institute) hydrolyzing α-1,6-glucoside bond in starch. Furthermore, as shown in Figure **14**, the glucoamylase-resistant component was partially decomposed with the single use of isoamylase and generated oligosaccharide with a degree of polymerization of 3 or more.

Under the circumstances described above, the glucoamylase-resistant component thus obtained was found to be cyclic glucan and to include at least one α-1,6-glucoside bond.

3-5: Structure of glucan obtained in Example 1

As evidence for the presence of an outer branched structure in the glucan obtained in Example 1, it was confirmed that glucosyl stubs were present in the glucoamylase-resistant component. Herein, the glucosyl stub refers to a glucose residue of a bound portion between an outer branched structure portion and a cyclic portion, which remains when the glucoamylase-resistant component is treated with glucoamylase. By determining non-reducing ends of the glucoamylase-resistant component by a rapid Smith degradation method described in Hizukuri et al. (1978) Carbohydr. Res: 63: 261-264, 19% of the entire glycosyl groups of the glucoamylase-resistant component were a glucosyl stub bound to a cyclic structure. Thus, it was considered that about 9 glucoses of the glucoamylase-resistant component (cyclic structure) having an average degree of polymerization of 49 were glucosyl stubs derived from the outer branched structure, and the remaining 40 glucoses formed an inner branched cyclic structure.

From the above, the glucan obtained in Example 1 was found to have a plurality of outer branched structures portions in addition to an inner branched cyclic structure. An outer branched structure portion is referred to as a set of the outer branched structures.

As described above, the glucan produced by the method of the present invention was found to have an outer branched structure portion and an inner branched cyclic structure portion and to have a degree of polymerization of about 50 or more. The glucan produced by the method of the present invention had an average degree of polymerization of about 900 and the inner branched structure portion thereof had an average degree of polymerization of about 40, so that the degree of polymerization of the outer branched structure portion was predicted to be about 860 in total. This suggests that the glucan produced by using branching enzyme according to the present invention has an inner branched cyclic structure portion and an outer branched structure portion in a ratio of about 1:20 on average.

(Example 4: Regulation of degree of polymerization of cyclic glucan)

First, 500 mg of the glucan with an average degree of polymerization of 900 obtained in Example 1 was thoroughly dissolved in 500 μl of DMSO. To this solution, 500 μl of 1 M sodium acetate buffer (pH 5.5), 3 ml of distilled water, and 1 ml of 10 units/ml of glucoamylase (Toyobo Co., Ltd.) were added, and the mixture was allowed to react at 40°C for 3 hours. The reaction solution was heated at 100°C for 10 minutes, and thereafter, denatured enzyme protein was removed by centrifugation at 10,000 rpm for 15 minutes. The amount of glucose in the supernatant was determined by the Glucostat method. It was found that about 70% glucosyl residue in the glucan obtained in Example 1 was deleted. Ten-fold volume of ethanol was added to the supernatant to precipitate glucan, whereby glucan having a shorter outer branched structure portion was obtained. The glucan thus obtained had an average degree of polymerization of 270.

C. Production method using D-enzyme (comparative Example)

(Example 5)

5-1: Production using D-enzyme

First, 40 mg of commercially available waxy corn starch was suspended in 2 ml of DMSO. Then, 18 ml of 20 mM citrate buffer (pH 7.0) containing 680 units of purified D-enzyme was added to the suspension, and the mixture was allowed to react at 30°C for 40 hours.

The reaction solution was heated at 100°C for 10 minutes and centrifuged to remove denatured enzyme protein. Then, 10-fold volume of ethanol was added to the supernatant to precipitate glucan. The precipitate was lyophilized to obtain about 40 mg of glucan powder.

The precipitate thus obtained was fractionated by gel filtration chromatography. The precipitate was dissolved in 250 μl of distilled water, and a total amount thereof was loaded onto a column obtained by connecting Superose 6 (ϕ1 cm x 30 cm, Pharmacia) to Superdex 30 (ϕ1 cm x 30 cm, Pharmacia). Then, elution was conducted at a flow rate of 1 ml/minute, using 150 mM of sodium acetate aqueous solution. As shown in Figure **15**, amylopectin eluted in a void volume was decomposed into lower molecules by the reaction of D-enzyme, and 2 kinds of components were generated, showing Peak I where an average molecular weight was about 30,000 and Peak II where an average molecular weight was about 3,000. The molecular weight of cyclic glucan is a value calculated using synthetic amylose as standards.

Fractions included in Peaks I and II were respectively collected. Ten-fold volume of ethanol was added to the fractions included in Peak I. Then, the precipitate was collected by centrifugation and lyophilized to obtain 20 mg of glucan. In the similar manner, the precipitate was collected from Peak II and lyophilized to obtain 5 mg of glucan .

Structures of glucans included in Peaks I and II thus separated were analyzed.

5-2: Structural analysis of glucans included in Peak I

The reducing end of the glucans included in Peak I was not detected, and the number of reducing ends did not increase, compared to the raw material, waxy corn starch.

Glucans included in Peak I were treated with glucoamylase under the same condition as that used in 3-2 of Example 3; and as a result, a glucoamylase-resistant component was obtained. The average molecular weight of the glucoamylase-resistant component was about 5,000, which was calculated by using synthetic amylose in the same manner as the above. This value was recognized to be smaller, compared with that before the glucoamylase treatment.

Furthermore, the glucoamylase-resistant component was digested with various kinds of amylases by the same method as that used in 3-4 of Example 3. As a result, the glucoamylase-resistant component was not decomposed by a single use of glucoamylase; however, it was decomposed with α-amylase, i.e., endo-type amylase which hydrolyzes an α-1,4-glucoside bond in a starch molecule, and IMM was detected. The glucoamylase-resistant component was also decomposed by the combined use of isoamylase and glucoamylase. Furthermore, the glucoamylase-resistant component was partially decomposed by a single use of isoamylase to generate oligosaccharide with a degree of polymerization of 3 or more.

From the above, the glucoamylase-resistant component thus obtained was found to have a cyclic structure containing at least one α-1,6-glucoside bond.

Furthermore, glucosyl stubs were confirmed to be present in the glucoamylase-resistant component. Thus, the glucans included in Peak I were concluded to be one having an inner branched cyclic structure portion and an outer branched structure portion.

5-3: Structural analysis of glucans included in Peak II

The reducing end of the glucans included in Peak II was not detected. Most of the glucans included in Peak II did not decrease in molecular weight, even when being treated with glucoamylase. The glucans included in Peak II were completely decomposed with α-amylase which is an endo-type amylase, and IMM was hardly detected. Furthermore, most of the glucans included in Peak II were not decomposed with isoamylase which hydrolyzes a α-1,6-glucoside bond.

From the above, the glucans included in Peak II were concluded to contain cyclic glucan not having a α-1,6-glucoside bond and having only α-1,4-glucoside bonds as its main component.

5-4: Glucan obtained in Example 5

It was concluded that the glucan obtained in Example 5 was a mixture of glucan having only α-1,4-glucoside bonds and glucan having an inner branched cyclic structure portion and an outer branched structure portion.

(Example 6: Structure of glucans included in Peak I of Example 5)

Glucans included in Peak I were proved to have an inner branched cyclic structure portion and an outer branched structure portion. The outer branched structure portion is completely decomposed into glucose with glucoamylase, and the inner branched cyclic structure portion (glucoamylase-resistant component) remains. Furthermore, the glucoamylase-resistant portion is decomposed with the combined use of debranching enzyme and glucoamylase. By utilizing these characteristics, it is possible to determine a non-cyclic structure portion in glucan, a cyclic structure portion containing an α-1,6-bond, and a cyclic structure portion containing no α-1,6-bond.

First, 10 mg of the glucans included in Peak I obtained in Example 5 and 10 mg of amylopectin were dissolved in 1 ml of DMSO separately, and the mixture was immediately diluted with 8 ml of 100 mM sodium acetate buffer (pH 5.0). Then, 900 μl aliquots of this dilution were dispensed into 4 tubes. Then, 100 μl of (1) distilled water, 100 μl of (2) a glucoamylase solution, 100 μl of (3) a mixed solution of debranching enzyme and glucoamylase, and 100 μl of (4) a mixed solution of endo-type α-amylase and glucoamylase were respectively added to the tubes, and the respective mixtures were allowed to react at 40°C for 4 hours. After the completion of reaction, the amount of glucose generated was measured by using a commercially available glucose measuring kit. The outer branched structure portion in the sample glucan, the inner branched cyclic structure portion, and the cyclic structure portion having only an α-1,4-glucoside bond were respectively calculated in accordance with the following formulae:

$$\text{Outer branched structure portion (\%)} = \frac{x - c}{z - c} \times 100$$

$$\text{Inner branched cyclic structure portion (\%)} = \frac{y - x}{z - c} \times 100$$

$$\text{Cyclic structure portion containing only } \alpha\text{-1,4-glucoside bonds (\%)} = \frac{z - y}{z - c} \times 100$$

where c, x, y, and z represent the amount of glucose generated from the above-mentioned reaction solutions (1), (2), (3), and (4), respectively. Table 1 shows the result.

Table 1

| | Waxy corn starch (%) | Glucans included in Peak I of Example 5 (%) |
|---|---|---|
| Outer branched structure portion | 98.9 | 86.9 |
| Cyclic portion containing no α-1,6-bond | 0.5 | 0.5 |
| Inner branched cyclic structure portion | 0.6 | 12.6 |

This result suggests that the glucans included in Peak I obtained in Example 5 contain at least one α-1,6-glucoside bond, and contain the inner branched cyclic structure portion and the outer branched structure portion in a ratio of about 1:7 on average.

D. Production of glucan using CGTase

(Example 7: Method for producing glucan, using CGTase)

As a raw material, 40 mg of commercially available waxy corn starch was dissolved with heating in 18 ml of 20 mM sodium acetate buffer (pH 5.5) containing 100 mM NaCl. Separately, purified CGTase was dissolved in 20 mM of sodium acetate buffer (pH 5.5) containing 100 mM of NaCl so as to be 0.2 units/ml. Then, 2 ml of this enzyme solution was added to the raw material solution, and the mixture was allowed to react at 40°C for 2 hours.

The reaction solution was heated at 100°C for 10 minutes, and denatured enzyme protein was removed by centrifugation. Ten-fold volume of acetone was added to the supernatant to precipitate glucan.

(Example 8: Structural analysis of glucan obtained in Example 7)

The precipitate thus obtained was subjected to gel filtration chromatography. The precipitate was dissolved in 250 μl of distilled water, and the entire amount of the solution was loaded onto connected columns of Superose 6 (ϕ1 cm x 30 cm, Pharmacia) and Superdex 30 (ϕ1 cm x 30 cm, Pharmacia). The elution was conducted with 20 mM of sodium acetate buffer (pH 5.5) containing 100 mM of NaCl at a flow rate of 1 ml/minute. As shown in Figure 16, amylopectin eluted in a void volume was decomposed into lower molecules by the reaction of CGTase, whereby 2 kinds of components included in Peaks III and IV were generated.

The glucans included in Peaks III and IV were fractionated, precipitated with acetone, collected, and lyophilized, whereby 16 mg and 13 mg of powders were respectively obtained.

The average molecular weight of the glucans included in Peak III was 30,000. The glucans included in Peak IV have a very low molecular weight; thus, they were not be able to be measured according to this method.

8-1 Structural analysis of glucans included in Peak III

Ten-fold volume of ethanol was added to the fractionated glucans included in Peak III to obtain a precipitate. The precipitate was collected by centrifugation and lyophilized, thereby obtaining 16 mg of powdery glucan.

The reducing end of the glucans included in Peak III was not detected, and the number of the reducing end did not increase, compared with the raw material, waxy corn starch.

The glucans included in Peak III were treated with glucoamylase under the same condition as that used in 3-2 of Example 3. As a result, a glucoamylase-resistant component was obtained. The average molecular weight of the glucoamylase-resistant component was about 5,000, which was calculated by using synthetic amylose in the same manner as the above. This value was recognized to be smaller, compared with that before the glucoamylase treatment.

Furthermore, the glucoamylase-resistant component was digested with various kinds of amylases by the same method as that used in 3-4 of Example 3. As a result, the glucoamylase-resistant component was not decomposed with the single use of glucoamylase; however, it was decomposed with α-amylase, i.e., endo-type amylase hydrolyzing an α-1,4-glucoside bond in a starch molecule, and IMM was detected. The glucoamylase-resistant component was also decomposed by the combination of isoamylase and glucoamylase. Furthermore, the glucoamylase-resistant component was partially decomposed with the single use of isoamylase to generate oligosaccharide with a degree of polymerization of 3 or more. Furthermore, glucosyl stubs were confirmed to be present in the glucoamylase-resistant component.

From the above, the glucoamylase-resistant component thus obtained was found to have a cyclic structure containing at least one α-1,6-glucoside bond.

Thus, the glucans included in Peak III were concluded to possess an inner branched cyclic structure portion and an outer branched structure portion.

8-2: Structural analysis of glucans included in Peak IV

The reducing end of the glucans included in Peak IV was not detected. The glucans included in Peak IV did not decrease in molecular weight, even when being treated with glucoamylase. The glucans included in Peak IV were not decomposed with isoamylase cutting an α-1,6-glucoside bond.

From the above, the glucans included in Peak IV were concluded to have a cyclic structure not having a α-1,6-glucoside bond or an outer branched structure, but having only α-1,4-glucoside bonds.

Separately, the glucans included in Peak IV were analyzed with HPLC using a column for sugars; and as a result, 2 peaks were confirmed. Each of the peaks were completely matched with the elution positions of cyclic α-1,4-glucan having a degree of polymerization of 6 and 7, i.e., α-CD and β-CD. Thus, the glucans included in Peak IV were concluded to be a mixture of the conventionally known α-CD and β-CD.

8-3 Structure of the glucan obtained in Example 7

From the above, the glucans obtained in Example 7 were concluded to be a mixture of α-CD and β-CD both having only α-1,4-glucoside bonds and glucan having an inner branched cyclic structure portion and an outer branched structure portion.

(Example 9: Structure of glucans included in Peak III of Example 7)

The inner branched cyclic structure portion and the outer branched structure portion of the glucans included in Peak III obtained in Example 7 were studied in the same way as in Example 6. Table 2 shows the result.

Table 2

| | Waxy corn starch (%) | Glucans included in Peak III of Example 7 (%) |
|---|---|---|
| Outer branched structure portion | 98.9 | 87.2 |
| Cyclic portion containing no α-1,6-bond | 0.5 | 0.1 |
| Inner branched cyclic structure portion | 0.6 | 12.7 |

This result shows that the glucans included in Peak III obtained in Example 7 and the glucans included in Peak I obtained in Example 5 are almost the same. That is, the results suggest that the glucans included in Peak III contain at least one α-1,6-glucoside bond and an inner branched cyclic structure portion and an outer branched structure portion in a ratio of about 1:7 on average.

It was proved from the above that glucan having an inner branched cyclic structure portion and an outer branched structure portion can be produced by using branching enzyme, D-enzyme, or CGTase, and almost the same glucan can be produced using different enzymes with similar activity.

(Example 10: Solubility of glucan obtained by the method of the present invention)

The glucan of Example 1, the glucan in Peak I of Example 5 and the glucan in Peak III of Example 7 were respectively suspended in distilled water so as to be 10% (w/v). For comparison, waxy corn starch and soluble starch (Wako Pure Chemical Industries, Ltd.) were also respectively suspended in distilled water so as to be 10% (w/v). The respective suspensions were vigorously stirred with a Vortex mixer and centrifuged at 12,000 rpm for 10 minutes to determine sugar concentration dissolved in the supernatant. Table 3 shows the result.

Table 3

| | Concentration of sugar dissolved in supernatant (%(w/v)) |
|---|---|
| Glucan of Example 1 | 10 |
| Peak I of Example 5 | 10 |
| Peak III of Example 7 | 10 |
| Soluble starch | 1.2 |
| Waxy corn starch | 0.8 |

Compared with the conventional waxy corn starch and soluble starch, it was found that the solubility of the glucans obtained by the method of the present invention was remarkably high.

(Example 11: Retrogradation of the glucan obtained in each example)

The glucan of Example 1, the glucan in Peak I of Example 5, the glucan included in Peak III of Example 7, the conventional waxy corn starch, and the conventional soluble starch (Wako Pure Chemical Industries, Ltd.) were respectively placed in screw vials in 200 mg portions. Then, 10 ml of distilled water was added to each screw vial, and each screw vial was heated in a hot water bath at about 100°C so as to obtain a solution. Here, since the waxy corn starch had poor solubility, 50 mg thereof was taken and treated in the same way. These solutions were allowed to stand at 4°C, and after a predetermined period of time, 1 ml each of sample was taken and the degree of suspension was measured at 660 nm as an index for retrogradation. Table 4 shows the result.

Table 4

| | Increase in turbidity at 660 nm | |
|---|---|---|
| | after 1 day | after 2 days |
| Glucan of Example 1 | 0 | 0 |
| Peak I of Example 5 | 0 | 0 |
| Peak III of Example 7 | 0 | 0 |
| Soluble starch | 0.182 | 0.583 |
| Waxy corn starch | 0.012 | 0.023 |

Compared with the conventional waxy corn starch and soluble starch, it was found that the retrogradation of the glucans obtained in the examples was remarkably low.

(Example 12: Viscosity of gelatinized solution of the glucans obtained in the examples)

The glucan of Example 1, the glucan in Peak I of Example 5, the glucan included in Peak III of Example 7, the conventional waxy corn starch, the conventional soluble starch (produced by Wako Pure Chemical Industries, Ltd.), and Pinedex #1 (Matsutani Kagaku Kabushikikaisha) were respectively placed in screw vials in 1.6 g portions. Then, 8 ml of distilled water was added to each screw vial to obtain a dispersion, 72 ml of dimethylsulfoxide was added to each dispersion, and each dispersion was thoroughly stirred at room temperature to obtain a solution. The viscosity of each solution was measured by a Digital Viscometer DVL-B (Rotor: No. 1, Rotation number: 60 rpm, measured for 10 seconds, manufactured by Tokyo Keiki). As a control, 8 ml of distilled water with 72 ml of dimethyl sulfoxide (90% DMSO solution) was used. The viscosity of each solution was measured in the same manner. Table 5 shows the result. Pinedex #1 used in the present example is starch more strongly hydrolyzed with α-amylase than the above-mentioned soluble starch.

Table 5

| | Viscosity (mPa·S) |
|---|---|
| 90% DMSO solution | 5.3 |
| Glucan of Example 1 | 6.4 |
| Peak I of Example 5 | 6.2 |
| Peak III of Example 7 | 6.1 |
| Pinedex #1 | 5.8 |
| Soluble starch | 5.6 |
| Waxy corn starch | 100.3 |

Compared with the conventional waxy corn starch, it was found that the viscosity of the gelatinized solution of the glucan obtained in each example was remarkably low and showed the similar viscosity to that of Pinedex #1 and the soluble starch.

(Example 13: Reactivity of the glucans obtained in the examples)

First, 20 mg each of the glucans of Example 1, in Peak I of Example 5 and in Peak III of Example 7, the conventional soluble starch and Pinedex #1 were respectively dissolved in 1 ml of distilled water. The reducing ability of each solution was determined by a dinitrosalicylic acid method (S. Fukui, Biochemical Experiment Method I, Assay of Reducing Sugar, Gakkai Shuppan Center) using glucose as a standard. Table 6 shows the result.

Table 6

| | Amount of reducing sugar (mg/ml) |
|---|---|
| Glucan of the present invention | Not detected (<0.01) |
| Peak I of Example 5 | Not detected (<0.01) |
| Peak III of Example 7 | Not detected (<0.01) |
| Pinedex #1 | 5.10 |
| Soluble starch | 2.84 |

Reducing sugar was not detected from the glucan obtained in each example, while reducing sugar was detected from the conventional Pinedex #1 and the soluble starch.

The color causing reaction which occurs between sugar and amino acid (i.e., amino-carbonyl reaction) is a reaction between a reducing group of sugar and an amino acid (O. Igarashi, Food Chemistry, Kogaku Shuppannsha). The glucan obtained in each example had less amount of reducing sugar (reducing sugar was not detected); in other words, the glucan had a lower amount of reducing groups. It was found from this fact that the glucan obtained in each example had low reactivity, compared with the conventional Pinedex #1 and the soluble starch.

(Example 14: Preparation of derivative of phosphorylated glucan)

First, 8 mg of the glucan obtained in Example 1 was suspended in 1 ml of dimethylformamide and allowed to react with 46 mg of phosphorus hydroxychloride, Then, 9 ml of acetone was added to the reaction solution to obtain 8 mg of derivative of phosphorylated glucan.

(Example 15: Effect of glucan of Example 1 on suppressing retrogradation starch)

When 4% soluble starch aqueous solution completely gelatinized by heating is kept at 4°C, the soluble starch rapidly retrogrades and the gelatinized solution becomes turbid. Using this system, the effect of suppressing the retrogradation of starch by the glucan obtained in the present invention was studied. The glucan obtained in Example 1 was added to 4% soluble starch aqueous solution so that the final concentration was 0%, 2%, and 4%, respectively, and the solutions were kept at 4°C. Each solution was sampled after a predetermined period of time, and the turbidity of each solution was measured at an absorbance of 660 nm. Figure 17 shows the result.

It was found that the glucan obtained in Example 1 had an effect of suppressing the retrogradation of starch.

(Example 16: Sports Drink containing the glucan of the present invention)

Sports drink containing the following components in the following proportion was prepared, using the glucan obtained in Example 1. It is noted that a unit is a part by weight.

| | |
|---|---|
| Salt | 0.3 |
| Vitamin C | 0.02 |
| Vitamin B1 | 0.02 |
| Magnesium chloride | 0.2 |
| Emulsified calcium | 0.2 |
| Citric acid | 2.0 |
| Sodium citrate | 1.5 |
| Glucose | 50 |
| Water | 1000 |
| Cyclic glucan of Example 1 | 100 |

The sports drink thus obtained had an outstanding digestive property and a high energy conversion efficiency.

(Example 17: Adhesive composition containing the glucan of the present invention)

Sixty parts of water were added to 40 parts of the glucan included in Peak I of Example 5, and the mixture was heated so that the glucan was thoroughly dissolved in water. The solution thus obtained showed satisfactory adhesion.

According to the method of the present invention, the glucan of the present invention can be produced using enzyme whose use has never been considered before. The step of allowing carbohydrate as a raw material having $\alpha$-1,4-glucoside bonds and at least one $\alpha$-1,6-glucoside bond to react with enzyme capable of acting on the carbohydrate to form a cyclic structure suffices. Thus, the glucan of the present invention can be very easily produced.

The glucan of the present invention has the following outstanding characteristics: the glucan has remarkably higher solubility in water, compared with the above-mentioned material such as existing starch; a gelatinized solution dissolving the glucan has low viscosity; and the glucan is not likely to retrograde, unlike conventional starch. Furthermore, the glucan of the present invention or derivatives thereof has low reactivity; therefore, when mixed with protein or amino acid and heated, the glucan is not likely to become colored, unlike the existing starch syrups and dextrin.

As described above, because of high solubility in water, the glucan of the present invention can be preferably used for powdered base, coffee, soy sauce, sauce for dipping noodles, Worcestershire sauce, broth stock, stew stock, soup stock, mixed seasonings, curry powder, jelly, caramel, chewing gum, chocolate, cookies, crackers, ice cream, sherbet, juice, powdery juice, a bathing agent, medicine for internal use, powdery medicine, paint, adhesive, thickener, and gelatinized material.

The glucan of the present invention is not likely to retrograde; therefore, it can be preferably used for Japanese confectionery, Western confectionery, frozen foods, cold foods, rice cakes, and rice balls.

Since a gelatinized solution dissolving the glucan of the present invention or derivatives thereof has low viscosity, the glucan or derivatives thereof can be preferably used as a raw material for biodegradable plastic, an intermediate material for producing cyclodextrin from starch, and a material in starch processing industry.

Furthermore, the glucan of the present invention has satisfactory adhesion; thus, it can be effectively used for an adhesive composition.

Still furthermore, the glucan of the present invention has some of the structure as conventional starch, except for a cyclic structure, so that it can be easily decomposed into glucose with enzymes in a living body, that is, the glucan has an outstanding digestive property. Therefore, the glucan of the present invention can be used for sports drinks, sports foods, etc.

Various other modifications will be apparent to and can be readily made by those skilled in the art without departing from the scope and spirit of this invention. Accordingly, it is not intended that the scope of the claims appended hereto be limited to the description as set forth herein, but rather that the claims be broadly construed.

(Sequence Listing)

SEQ ID NO: 1

SEQUENCE LENGTH: 21 base pairs
SEQUENCE TYPE: nucleic acid
STRANDEDNESS: single
TOPOLOGY: linear
SEQUENCE TYPE: other nucleic acid synthetic DNA

SEQUENCE: 5' GAYTG GGTNC CNGSN CAYTT Y 3' 21

SEQ ID NO: 2

SEQUENCE LENGTH: 21 base pairs
SEQUENCE TYPE: nucleic acid
STRANDEDNESS: single
TOPOLOGY: linear
SEQUENCE TYPE: other nucleic acid synthetic DNA

SEQUENCE: 5' RTGNACNACYTCRTCRTGNSW 3' 21

SEQ ID NO: 3

SEQUENCE LENGTH: 2426 base pairs
SEQUENCE TYPE: nucleic acid
STRANDEDNESS: double
TOPOLOGY: linear
SEQUENCE TYPE: genomic DNA
ORIGINAL SOURCE

ORGANISM: Bacillus stearothermophilus
STRAIN NAME: TRBE14

FEATURE 385-392 S SD sequence
402-2357 P CDS

SEQUENCE

```
AGCGCTAAGA.CATCGGAAGC CATATGGGCC GAATCGGACA ACAAAGCGAG CGAGTTCGAG    60
AGCACCCCAC CGATGATTTC AACAACCGTG AAAAAGACGG TGAGCACGAG TGTAATCCAA   120
AGCGCCTTTT TCGACTGGGT TTGTGTTTTC ACGTGTGGAA GATGATGAAA ATCGTATGCT   180
CCAGGAGACA TAATACACCT CTTTTTTATA ATAATTATTA ATTTATATCT ATTAAAATAT   240
AAAAAGTGCT AGGTGTGCAA AAAATTTATC GACAAATCCC ACAATTTTTG ATGGATTTTG   300
TCAAAAAATA TAGATAATTT TTTCGAGGAA TGCAGCGGTG AAGTGGCGAA TAAGGAATAA   360
TGTGTGGCCA TCATCTTGCT TTGGAAAGGA TGCGATACGG T TTG ATT GCG GCG AAT   416
                                              Met Ile Ala Ala Asn
                                               1               5
CCG ACG GAT TTG GAA GTG TAT TTG TTT CAT GAA GGC AGC TTG TAT AAA     464
Pro Thr Asp Leu Glu Val Tyr Leu Phe His Glu Gly Ser Leu Tyr Lys
                10                  15                  20
AGT TAC GAG CTG TTT GGC GCC CAT GTG ATT AAT GAG GGC GGG AAG GTC     512
Ser Tyr Glu Leu Phe Gly Ala His Val Ile Asn Glu Gly Gly Lys Val
            25                  30                  35
GGC ACC CGT TTT TGT GTT TGG GCG CCG CAC GCG CGC GAG GTG CGT CTT     560
Gly Thr Arg Phe Cys Val Trp Ala Pro His Ala Arg Glu Val Arg Leu
        40                  45                  50
GTC GGC AGT TTC AAC GAT TGG GAC GGG ACG GAT TTT CGC CTT GAG AAA     608
Val Gly Ser Phe Asn Asp Trp Asp Gly Thr Asp Phe Arg Leu Glu Lys
    55                  60                  65
GTG AAT GAT GAA GGG GTA TGG ACG ATT GTT GTC CCC GAA AAC TTG GAA     656
Val Asn Asp Glu Gly Val Trp Thr Ile Val Val Pro Glu Asn Leu Glu
70                  75                  80                  85
GGG CAT TTA TAT AAG TAT GAG ATT GTT ACG CCG GAC GGA CAG GTG CTG     704
Gly His Leu Tyr Lys Tyr Glu Ile Val Thr Pro Asp Gly Gln Val Leu
                90                  95                  100
```

```
TTC AAA GCC GAC CCG TAC GCT TTT TAC TCC GAA TTG CGT CCT CAT ACC      752
Phe Lys Ala Asp Pro Tyr Ala Phe Tyr Ser Glu Leu Arg Pro His Thr
            105                 110                 115
GCC TCG ATT GCC TAC GAT CTG AAA GGA TAC CAG TGG AAC GAT CAA TCT      800
Ala Ser Ile Ala Tyr Asp Leu Lys Gly Tyr Gln Trp Asn Asp Gln Ser
        120                 125                 130
TGG AAG CGG AAG AAG CGA CGA AAA CGG ATT TAT GAT CAG CCC ATG GTG      848
Trp Lys Arg Lys Lys Arg Arg Lys Arg Ile Tyr Asp Gln Pro Met Val
    135                 140                 145
ATT TAT GAA CTC CAT TTC GGT TCG TGG AAG AAA AAA GAT GGG CGT TTT      896
Ile Tyr Glu Leu His Phe Gly Ser Trp Lys Lys Lys Asp Gly Arg Phe
150                 155                 160                 165
TAT ACG TAC CGT GAG ATG GCC GAT GAA CTG ATC TCG TAT GTG CTC GAT      944
Tyr Thr Tyr Arg Glu Met Ala Asp Glu Leu Ile Ser Tyr Val Leu Asp
                170                 175                 180
CAT GGG TTT ACG CAC ATT GAG TTG CTT CCT CTC GTC GAG CAT CCG CTC      992
His Gly Phe Thr His Ile Glu Leu Leu Pro Leu Val Glu His Pro Leu
            185                 190                 195
GAC CGC TCG TGG GGC TAT CAA GGA ACA GGG TAT TAT GCG GTA ACG AGT     1040
Asp Arg Ser Trp Gly Tyr Gln Gly Thr Gly Tyr Tyr Ala Val Thr Ser
        200                 205                 210
CGC TAT GGT ACG CCA CAC GAC TTC ATG TAC TTC GTC GAC CGT TGC CAT     1088
Arg Tyr Gly Thr Pro His Asp Phe Met Tyr Phe Val Asp Arg Cys His
    215                 220                 225
CAG GCG GGA ATC GGG GTC ATT ATG GAC TGG GTG CCG GGC CAT TTT TGC     1136
Gln Ala Gly Ile Gly Val Ile Met Asp Trp Val Pro Gly His Phe Cys
230                 235                 240                 250
AAG GAC GCC CAT GGG TTA TAT ATG TTT GAT GGC GCC CCG ACG TAT GAA     1184
Lys Asp Ala His Gly Leu Tyr Met Phe Asp Gly Ala Pro Thr Tyr Glu
```

```
                255                 260                 265
TAC GCG AAT GAA AAA GAC CGA GAA AAT TAC GTT TGG GGG ACG GCC AAT   1232
Tyr Ala Asn Glu Lys Asp Arg Glu Asn Tyr Val Trp Gly Thr Ala Asn
            270                 275                 280
TTT GAT TTA GGC AAG CCG GAA GTG CGC AGT TTT CTC ATC TCG AAC GCA   1280
Phe Asp Leu Gly Lys Pro Glu Val Arg Ser Phe Leu Ile Ser Asn Ala
        285                 290                 295
TTG TTT TGG CTC GAG TAT TAC CAT ATC GAC GGG TTC CGG GTC GAT GCG   1328
Leu Phe Trp Leu Glu Tyr Tyr His Ile Asp Gly Phe Arg Val Asp Ala
    300                 305                 310
GTT GCC AAT ATG CTT TAT TGG CCG AAC AAT GAC AGG CTG TAC GAG AAC   1376
Val Ala Asn Met Leu Tyr Trp Pro Asn Asn Asp Arg Leu Tyr Glu Asn
315                 320                 325                 330
CCG TAT GCG GTC GAG TTT TTG CGC AAG TTA AAC GAA GCG GTG TTT GCC   1424
Pro Tyr Ala Val Glu Phe Leu Arg Lys Leu Asn Glu Ala Val Phe Ala
                335                 340                 345
TAT GAT CCG AAT GCG CTG ATG ATT GCG GAA GAT TCG ACT GAC TGG CCG   1472
Tyr Asp Pro Asn Ala Leu Met Ile Ala Glu Asp Ser Thr Asp Trp Pro
            350                 355                 360
AAG GTG ACC GCG CCG ACG TAT GAA GGC GGA CTC GGC TTT AAT TAT AAA   1520
Lys Val Thr Ala Pro Thr Tyr Glu Gly Gly Leu Gly Phe Asn Tyr Lys
        365                 370                 375
TGG AAC ATG GGC TGG ATG AAC GAC ATG CTG AAG TAC ATG GAA ACA CCG   1568
Trp Asn Met Gly Trp Met Asn Asp Met Leu Lys Tyr Met Glu Thr Pro
    380                 385                 390
CCG TAT GAG CGG AGG CAT GTG CAT AAC CAA GTA ACG TTC TCC CTC CTT   1616
Pro Tyr Glu Arg Arg His Val His Asn Gln Val Thr Phe Ser Leu Leu
390                 395                 400                 405
TAT GCG TAT TCG GAA AAT TTC ATT TTG CCG TTT TCC CAC GAT GAA GTC   1664
```

Tyr Ala Tyr Ser Glu Asn Phe Ile Leu Pro Phe Ser His Asp Glu Val
410 415 420

GTG CAT GGC AAA AAA TCG CTG CTC AAT AAA ATG CCA GGG TCG TAT GAA 1712
Val His Gly Lys Lys Ser Leu Leu Asn Lys Met Pro Gly Ser Tyr Glu
425 430 435

GAG AAG TTC GCC CAG CTG CGC CTC TTG TAC GGC TAC ATG ATG GCT CAT 1760
Glu Lys Phe Ala Gln Leu Arg Leu Leu Tyr Gly Tyr Met Met Ala His
440 445 450

CCG GGG AAA AAG CTG TTG TTT ATG GGC AAT GAA TTT GCT CAG TTT GAT 1808
Pro Gly Lys Lys Leu Leu Phe Met Gly Asn Glu Phe Ala Gln Phe Asp
455 460 465

GAA TGG AAG TTT GAG GAT GAA CTC GAT TGG GTG CTG TTT GAT TTT GAG 1856
Glu Trp Lys Phe Glu Asp Glu Leu Asp Trp Val Leu Phe Asp Phe Glu
470 475 480 485

CTG CAC CGG AAG ATG AAC GAT TAC ATG AAA GAG TTA ATC GCC TGC TAT 1904
Leu His Arg Lys Met Asn Asp Tyr Met Lys Glu Leu Ile Ala Cys Tyr
490 495 500

AAA CGG TAT AAG CCG TTT TAC GAA TTG GAT CAT GAC CCG CAA GGA TTT 1952
Lys Arg Tyr Lys Pro Phe Tyr Glu Leu Asp His Asp Pro Gln Gly Phe
505 510 515

GAA TGG ATT GAC GTT CAC AAC GCT GAA CAA AGC ATT TTC TCA TTC ATC 2000
Glu Trp Ile Asp Val His Asn Ala Glu Gln Ser Ile Phe Ser Phe Ile
520 525 530

CGC CGC GGG AAA AAA GAA GAT GAT GTG CTT GTT ATT GTT TGC AAT TTC 2048
Arg Arg Gly Lys Lys Glu Asp Asp Val Leu Val Ile Val Cys Asn Phe
535 540 545

ACA AAT CAG GCG TAT GAC GAC TAC AAA GTT GGA GTG CCG TTG CTC GTA 2096
Thr Asn Gln Ala Tyr Asp Asp Tyr Lys Val Gly Val Pro Leu Leu Val
550 555 560 565

```
CCG TAT CGG GAA GTG CTG AAT AGC GAT GCG GTC ACG TTT GGT GGA TCG      2144
Pro Tyr Arg Glu Val Leu Asn Ser Asp Ala Val Thr Phe Gly Gly Ser
                570                 575                 580
GGG CAT GTC AAT GGG AAA CGG CTT TCC GCC TTC AAT GAG CCG TTT CAT      2192
Gly His Val Asn Gly Lys Arg Leu Ser Ala Phe Asn Glu Pro Phe His
            585                 590                 595
GGT AAA CCA TAC CAC GTG CGC ATG ACG ATT CCG CCA TTT GGC ATT TCC      2240
Gly Lys Pro Tyr His Val Arg Met Thr Ile Pro Pro Phe Gly Ile Ser
        600                 605                 610
ATT TTA CGG CCA GTG CAA AAA CGA GGG GAG AGA AAG CGA AAT GAA AAA      2288
Ile Leu Arg Pro Val Gln Lys Arg Gly Glu Arg Lys Arg Asn Glu Lys
    615                 620                 625
GAA ATG CAT CGC CAT GTT ATT GGC CGG CGG GCA AGG AAG TCG GCT TCG      2336
Glu Met His Arg His Val Ile Gly Arg Arg Ala Arg Lys Ser Ala Ser
630                 635                 640                 645
CTC GCT GAC GAC AAA CAT CGC TAAACCGGCC GTGCCATTCG GCGGGAAGTA        2387
Leu Ala Asp Asp Lys His Arg
                650
CCGGATCATT GATTTTACAT TAAGCAATTG CACGAATTC                          2426
```

## Claims

1. A method for producing glucan, comprising the step of:

   allowing a carbohydrate containing α-1,4-glucoside bonds and at least one α-1,6-glucoside bond to react with branching enzyme so as to generate glucan having a degree of polymerization of 50 or more, whose molecular weight is smaller than the molecular weight of the carbohydrate, and including an inner branched cyclic structure portion and an outer branched structure portion,

   wherein the inner branched cyclic structure portion is a cyclic structure portion formed from α-1,4-glucoside bonds and at least one α-1,6-glucoside bond and wherein the outer branched structure portion is a non-cyclic structure portion bound to the inner branched cyclic structure portion.

2. The method according to claim 1, wherein the carbohydrate is starch.

**3.** The method according to claim 1, wherein the carbohydrate is amylopectin.

**4.** The method according to claim 1, wherein the degree of polymerization of the glucan is in the range of 50 to 5,000.

**5.** The method according to claim 1, wherein a degree of polymerization of the inner branched cyclic structure portion of the glucan is in the range of 10 to 100.

**6.** The method according to claim 1, wherein a degree of polymerization of the outer branched structure portion of the glucan is 40 or more.

**7.** The method according to claim 6, wherein a degree of polymerization of each unit chain of the outer branched structure portion of the glucan is in the range of 10 to 20 on an average.

**8.** The method according to claim 1, wherein said reaction step is conducted under conditions including pH of from 3 to 11, temperature of from 10°C to 110°C and a substrate concentration of from 0.05% to 60% by weight.

**9.** The method according to claim 1, wherein the amount of the enzyme is in the range of 50 to 10,000 units per gram of substrate.

**10.** The method according to claim 1, further comprising the step of purifying the glucan formed in the reacting steps.

**11.** A method for producing glucan, comprising the step of:

allowing a carbohydrate having only α-1,4-glucoside bonds to react with branching enzyme so as to generate glucan having a degree of polymerization of 50 or more, whose molecular weight is smaller than the molecular weight of the carbohydrate, and including an inner branched cyclic structure portion and an outer branched structure portion,

wherein the inner branched cyclic structure portion is a cyclic structure portion formed from α-1,4-glucoside bonds and at least one α-1,6-glucoside bond and wherein the outer branched structure portion is a non-cyclic structure portion bound to the inner branched cyclic structure portion.

**Patentansprüche**

**1.** Verfahren zur Herstellung eines Glucans, Schritte umfassend, bei denen man:

ein Kohlenhydrat, das α-1,4-glykosidische Bindungen und mindestens eine α-1,6-glykosidische Bindung aufweist, mit Branching-Enzym umsetzen läßt, wobei Glucan erzeugt wird, welcher einen Polymerisierungsgrad von 50 oder mehr aufweist, dessen Molekulargewicht kleiner als das Molekulargewicht des Kohlenhydrats ist und der einen inneren, verzweigten, cyclischen Struktur-Teil und einen äußeren, verzweigten Struktur-Teil umfaßt,

worin der innere, verzweigte, cyclische Struktur-Teil ein aus α-1,4-glykosidischen Bindungen und mindestens einer α-1,6-glykosidischen Bindung gebildeter, cyclischer Struktur-Teil ist, und worin der äußere, verzweigte Struktur-Teil einen an den inneren, verzweigten cyclischen Struktur-Teil gebundenen nicht-cyclischen Struktur-Teil aufweist.

**2.** Verfahren gemäß Anspruch 1, bei dem das Kohlenhydrat Stärke ist.

**3.** Verfahren gemäß Anspruch 1, bei dem das Kohlenhydrat Amylopektin ist.

**4.** Verfahren gemäß Anspruch 1, bei dem der Polymerisierungsgrad des Glucans im Bereich von 50 bis 5000 liegt.

**5.** Verfahren gemäß Anspruch 1, bei dem der Polymerisierungsgrad des inneren, verzweigten, cyclischen Struktur-Teils des Glucans im Bereich von 10 bis 100 liegt.

**6.** Verfahren gemäß Anspruch 1, bei dem der Polymerisierungsgrad des äußeren, verzweigten Struktur-Teils des Glucans 40 oder mehr beträgt.

7. Verfahren gemäß Anspruch 6, bei dem der Polymerisierungsgrad jeder Ketteneinheit des äußeren, verzweigten Struktur-Teils des Glucans durchschnittlich im Bereich von 10 bis 20 liegt.

8. Verfahren gemäß Anspruch 1, bei dem der Reaktionsschritt unter Bedingungen durchgeführt wird, die einen pH-Wert von 3 bis 11, eine Temperatur von 10 °C bis 110 °C und eine Substratkonzentration von 0,05 % bis 60 % in Bezug auf das Gewicht umfassen.

9. Verfahren gemäß Anspruch 1, bei dem die Menge des Enzyms in dem Bereich von 50 bis 10 000 Einheiten pro Gramm Substrat liegt.

10. Verfahren gemäß Anspruch 1, welches ferner den Schritt der Reinigung des in dem Reaktionsschritt gebildeten Glucans umfasst.

11. Verfahren zur Herstellung eines Glucans, Schritte umfassend, bei denen man:

ein Kohlenhydrat mit nur α-1,4-glykosidischen Bindungen mit Branching-Enzym umsetzt, wodurch ein Glucan mit einem Polymerisationsgrad von 50 oder mehr erzeugt wird, dessen Molekulargewicht kleiner als das Molekulargewicht des Kohlenhydrates ist und der einen inneren, verzweigten, cyclischen Struktur-Teil und einen äußeren, verzweigten Struktur-Teil umfasst,

worin der innere, verzweigte, cyclische Struktur-Teil eine aus α-1,4-glykosidischen Bindungen und mindestens einer α-1,6-glykosidischen Bindung gebildete cyclische Struktur aufweist und der äußere, verzweigte Struktur-Teil ein nicht-cyclischer Struktur-Teil ist, welcher an den inneren, verzweigten, cyclischen Struktur-Teil gebunden ist.

**Revendications**

1. Procédé pour produire un glucane, comprenant l'étape consistant :

à laisser un sucre contenant des liaisons α-1,4-glucoside et au moins une liaison α-1,6-glucoside réagir avec une enzyme de ramification de façon à engendrer un glucane ayant un degré de polymérisation de 50 ou plus, dont la masse moléculaire est inférieure à la masse moléculaire du sucre, et incluant une partie de structure cyclique à ramification interne et une partie de structure à ramification externe,

dans lequel la partie de structure cyclique à ramification interne est une partie de structure cyclique formée à partir des liaisons α-1,4-glucoside et d'au moins une liaison α-1,6-glucoside et dans lequel la partie de structure à ramification externe est une partie de structure non cyclique liée à la partie de structure cyclique à ramification interne.

2. Procédé selon la revendication 1, dans lequel le sucre est l'amidon.

3. Procédé selon la revendication 1, dans lequel le sucre est l'amylopectine.

4. Procédé selon la revendication 1, dans lequel le degré de polymérisation du glucane est dans la gamme de 50 à 5 000.

5. Procédé selon la revendication 1, dans lequel un degré de polymérisation de la partie de structure cyclique à ramification interne du glucane est dans la gamme de 10 à 100.

6. Procédé selon la revendication 1, dans lequel un degré de polymérisation de la partie de structure à ramification externe du glucane est de 40 ou plus.

7. Procédé selon la revendication 6, dans lequel un degré de polymérisation de chaque chaîne unitaire de la partie de structure à ramification externe du glucane est dans la gamme de 10 à 20 en moyenne.

8. Procédé selon la revendication 1, dans lequel ladite étape de réaction est réalisée dans des conditions comprenant un pH allant de 3 à 11, une température allant de 10°C à 110°C et une concentration en substrat allant de 0,05 % à 60 % en poids.

**9.** Procédé selon la revendication 1, dans lequel la quantité de l'enzyme est dans la gamme de 50 à 10 000 unités par gramme de substrat.

**10.** Procédé selon la revendication 1, comprenant l'étape supplémentaire de purification du glucane formé dans les étapes de réaction.

**11.** Procédé pour produire un glucane, comprenant l'étape consistant :

à laisser un sucre n'ayant que des liaisons α-1,4-glucoside réagir avec une enzyme de ramification de façon à engendrer un glucane ayant un degré de polymérisation de 50 ou plus, dont la masse moléculaire est inférieure à la masse moléculaire du sucre, et comprenant une partie de structure cyclique à ramification interne et une partie de structure à ramification externe,

dans lequel la partie de structure cyclique à ramification interne est une partie de structure cyclique formée à partir des liaisons α-1,4-glucoside et d'au moins une liaison α-1,6-glucoside et dans lequel la partie de structure à ramification externe est une partie de structure non cyclique liée à la partie de structure cyclique à ramification interne.

FIG.1

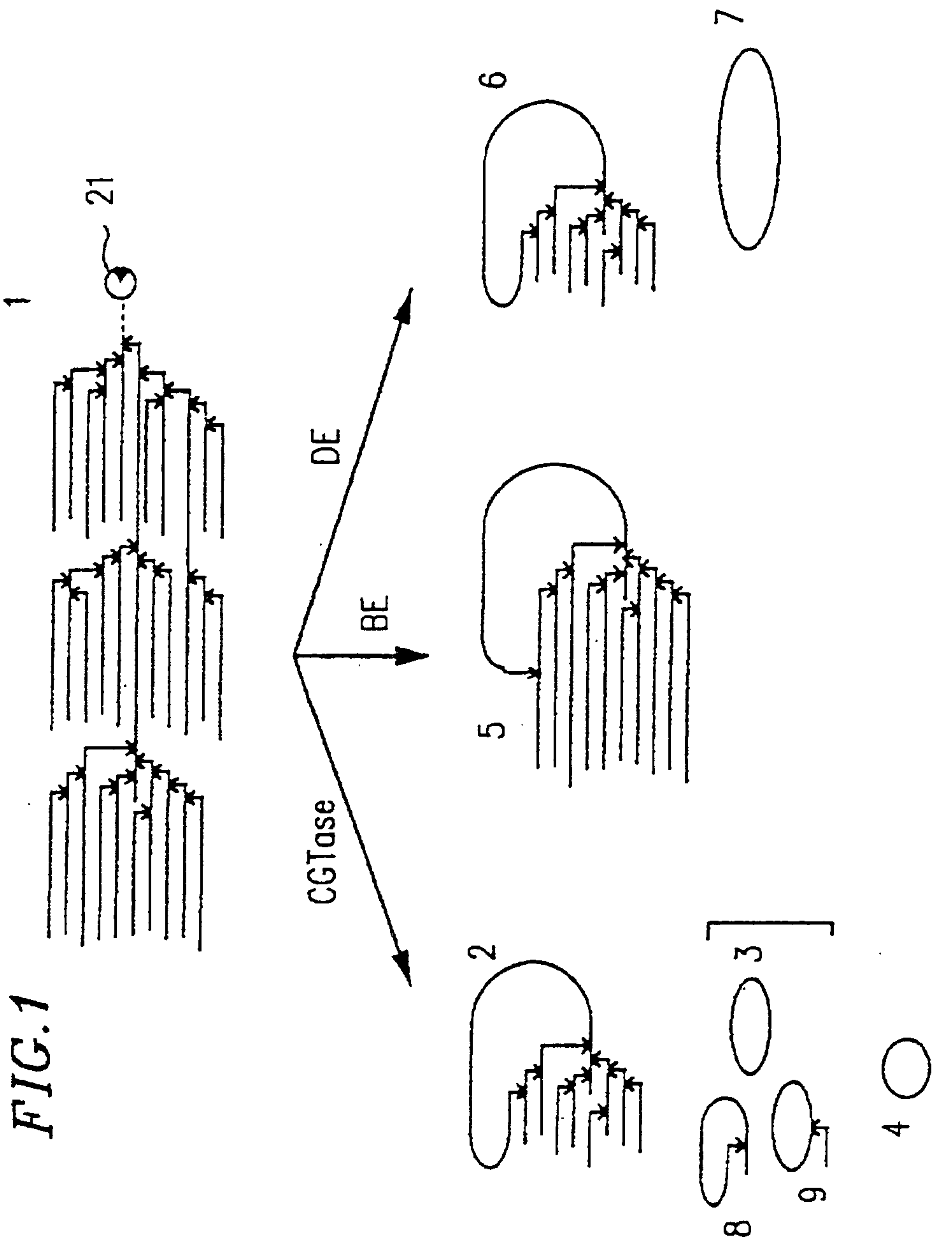

*FIG. 2*

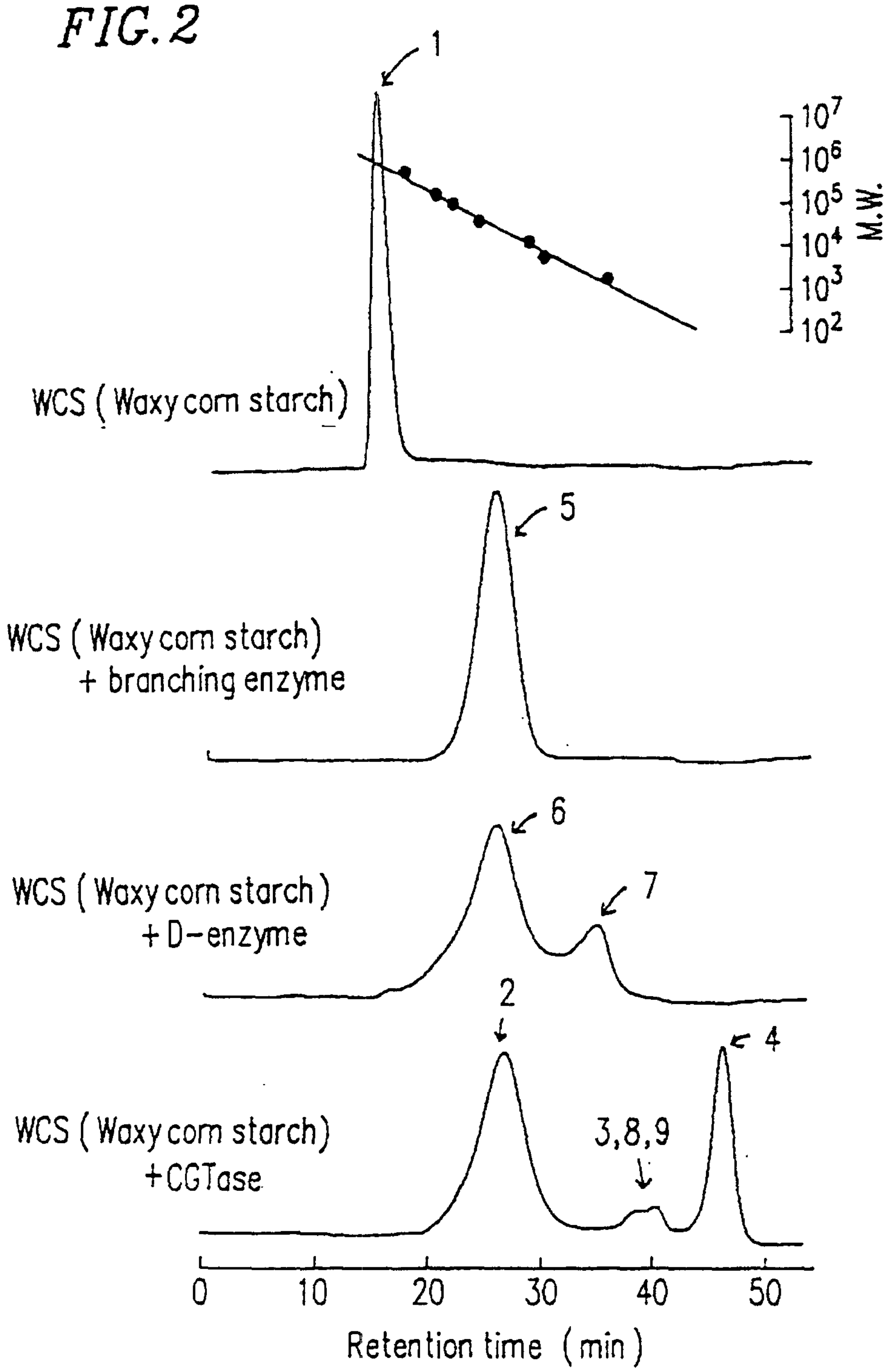
1
10^7
10^6
10^5
10^4
10^3
10^2
M.W.
WCS ( Waxy corn starch)
5
WCS ( Waxy corn starch)
+ branching enzyme
6
7
WCS ( Waxy corn starch)
+ D-enzyme
2
4
WCS ( Waxy corn starch)
+ CGTase
3,8,9
0
10
20
30
40
50
Retention time (min)

FIG. 3

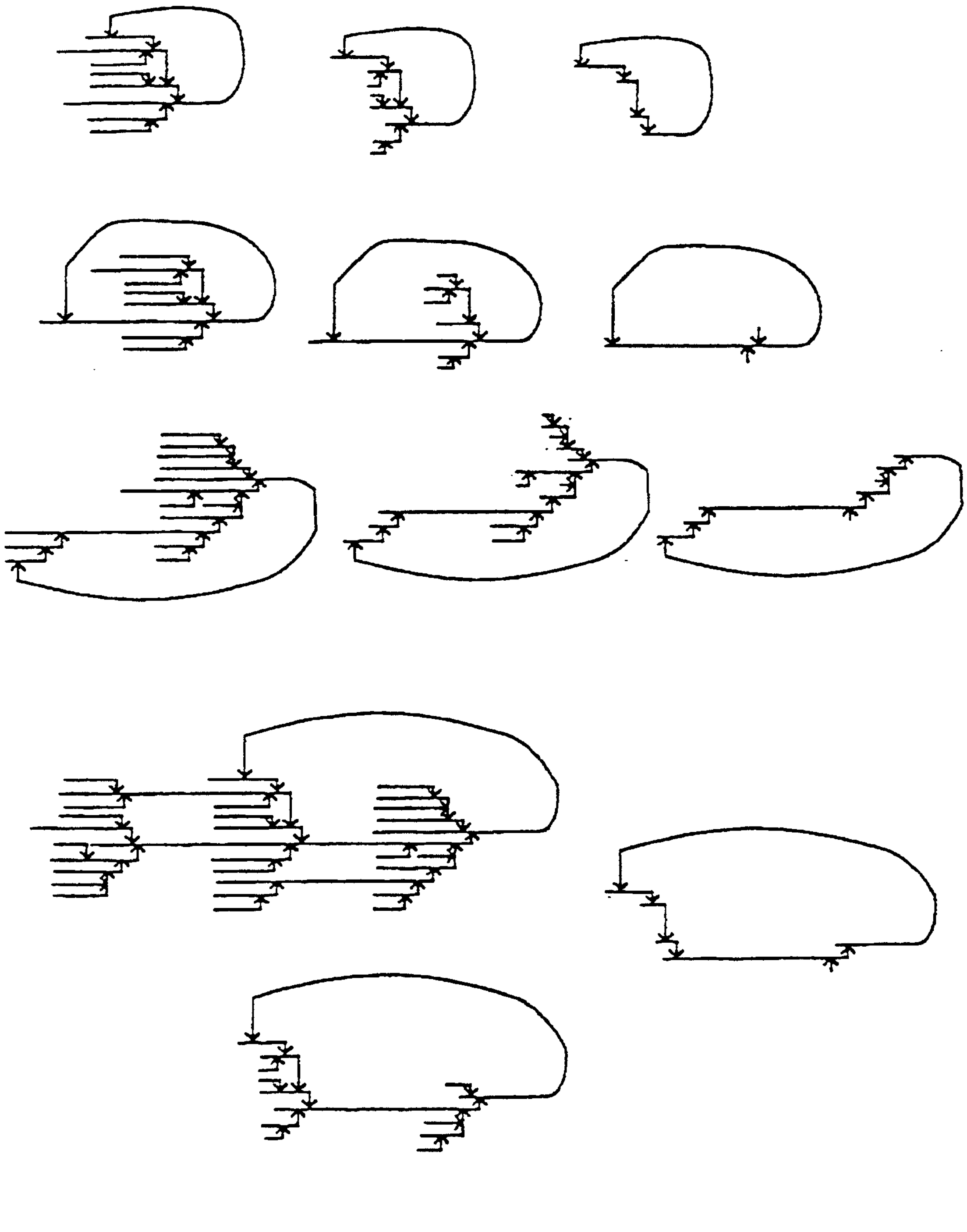

FIG. 4B

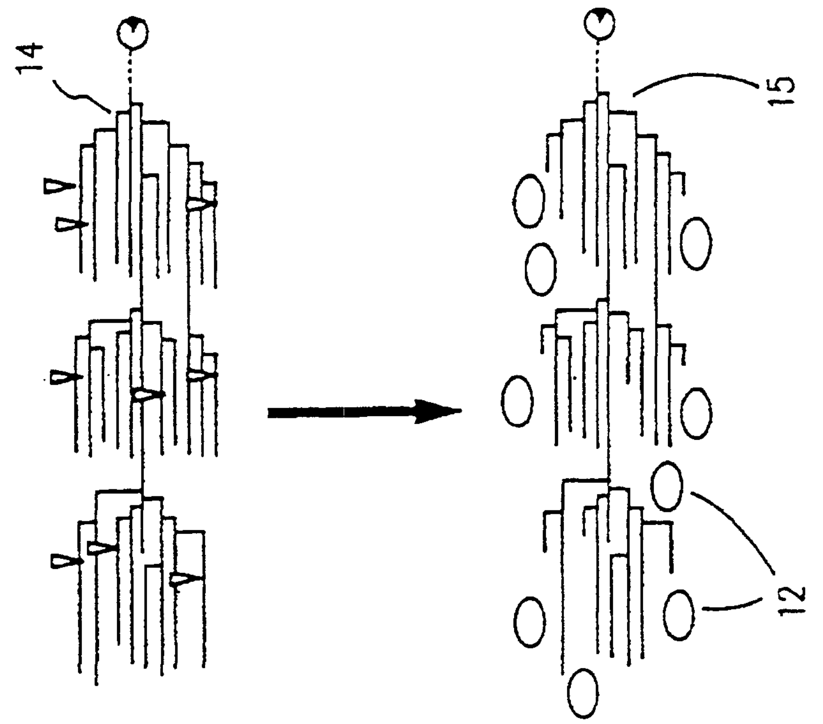


FIG. 4A

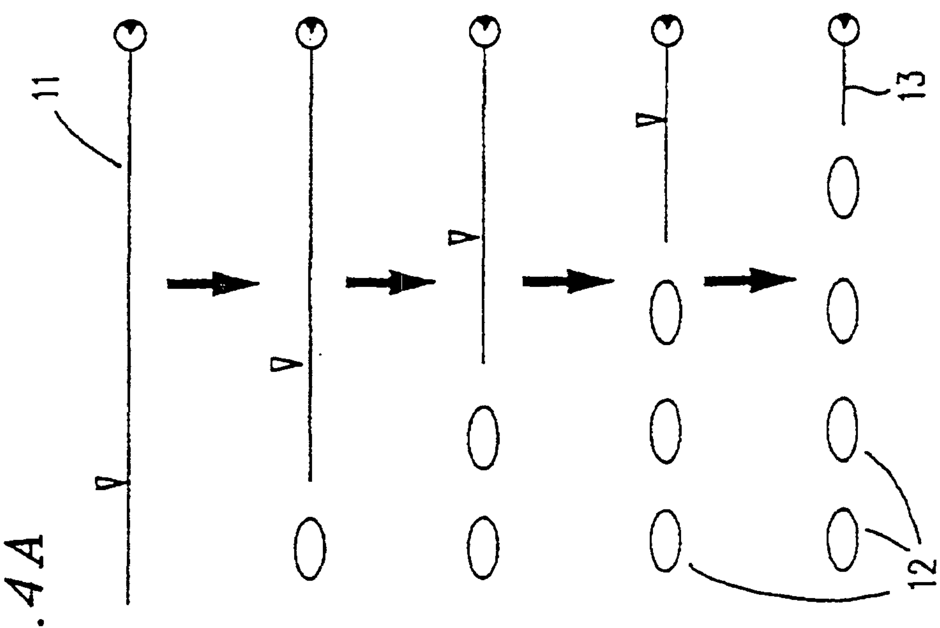

*FIG. 5*

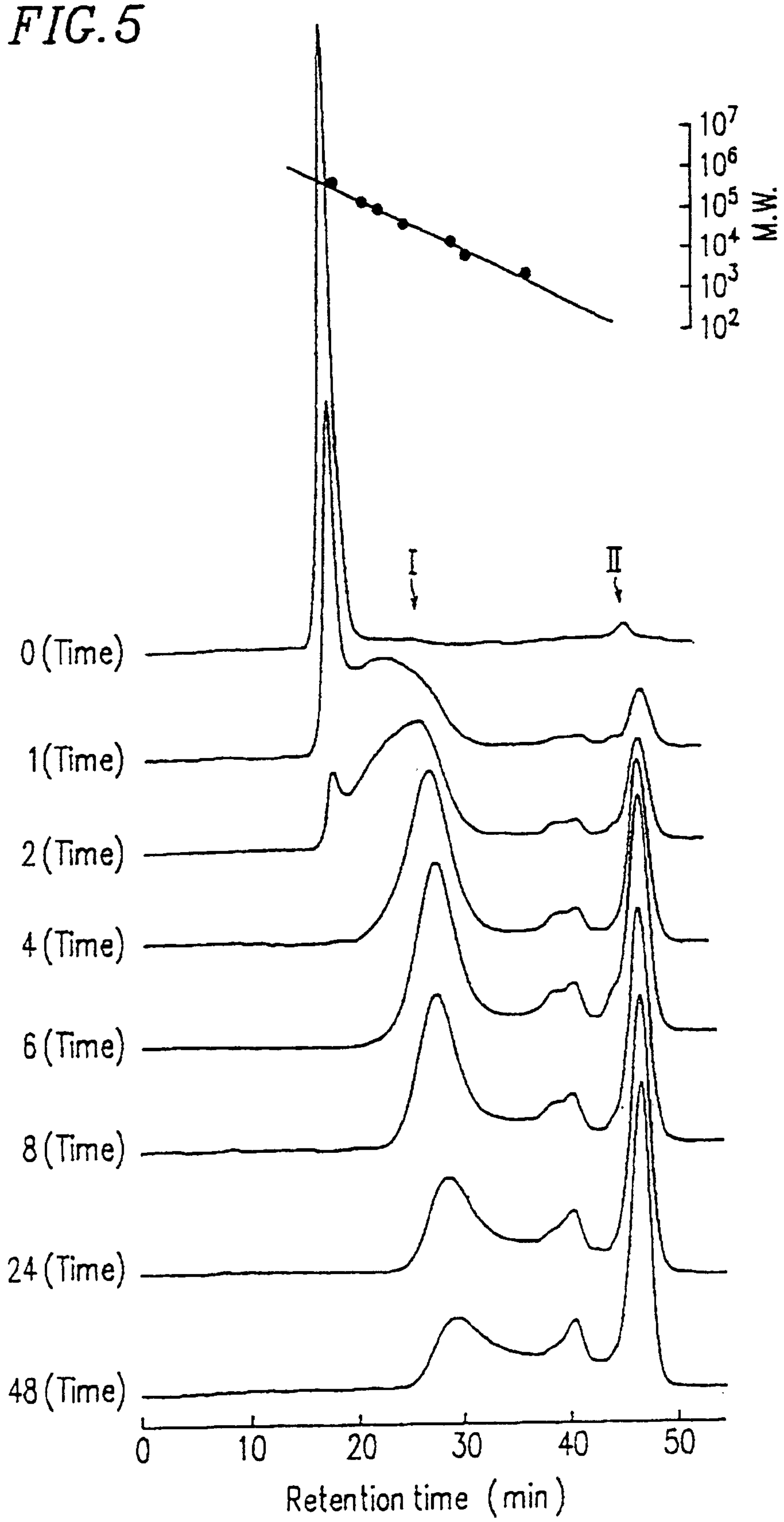
$10^7$
$10^6$
$10^5$
$10^4$
$10^3$
$10^2$
M.W.
I
II
0 (Time)
1 (Time)
2 (Time)
4 (Time)
6 (Time)
8 (Time)
24 (Time)
48 (Time)
0
10
20
30
40
50
Retention time (min)

FIG.6

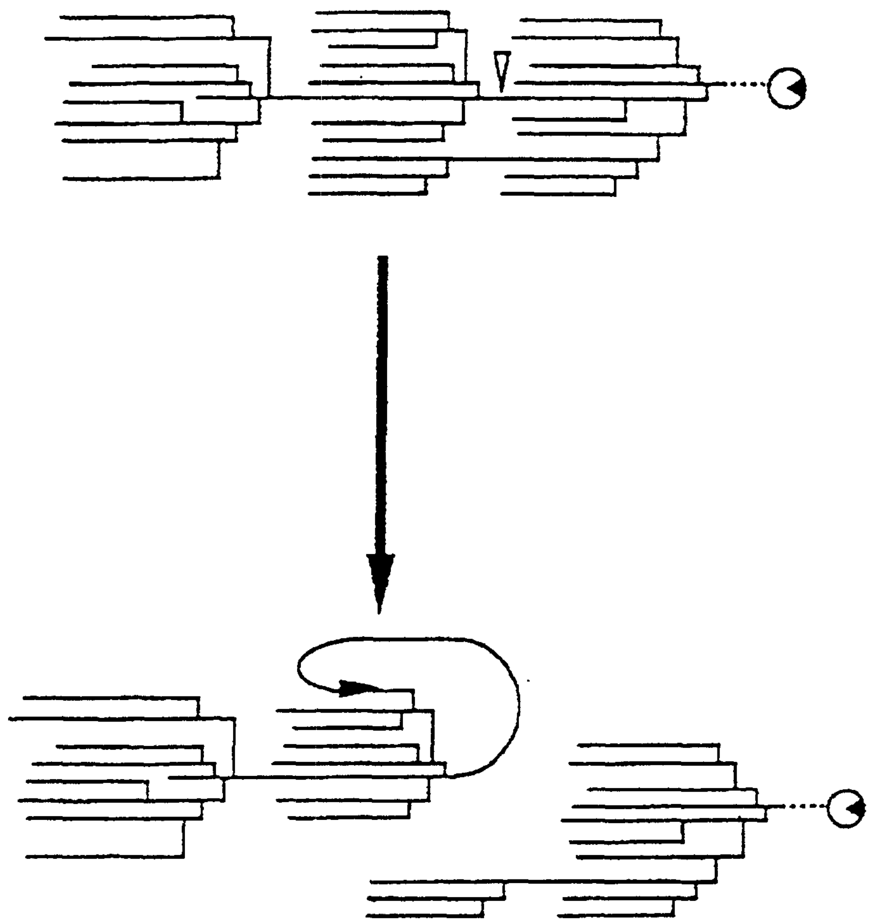

*FIG.7*

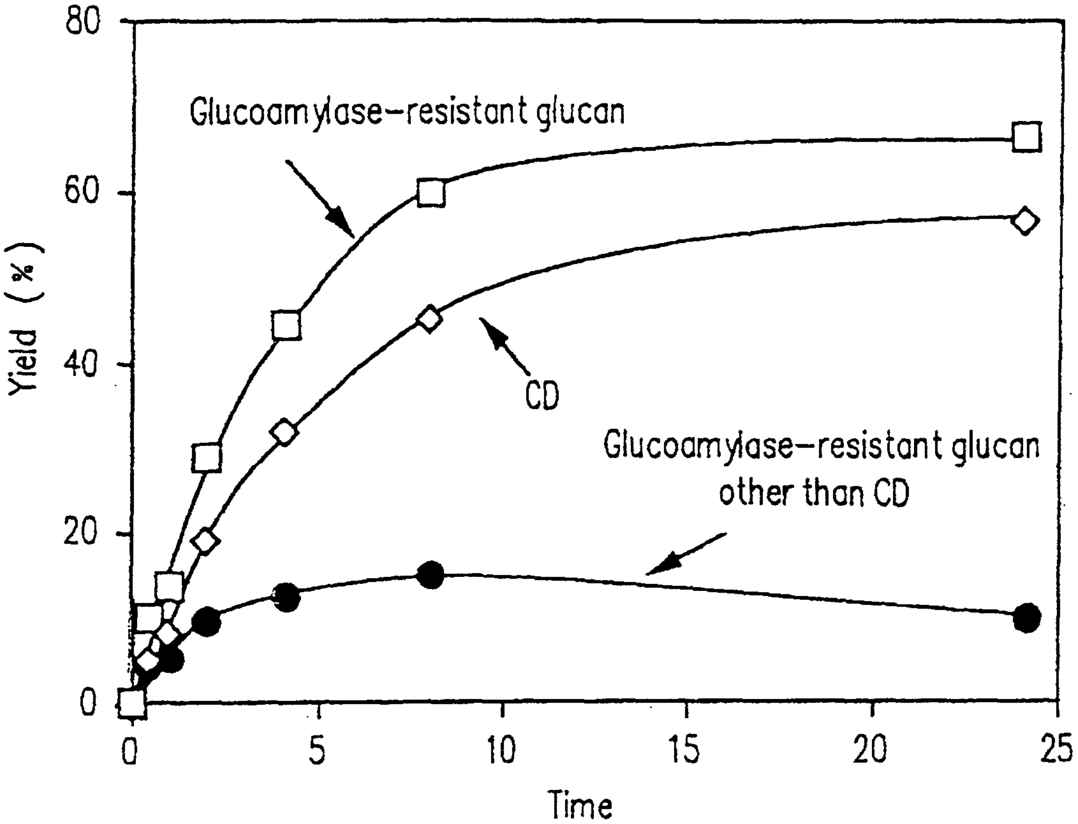
80
60
40
20
0
Yield (%)
0
5
10
15
20
25
Time
Glucoamylase-resistant glucan
CD
Glucoamylase-resistant glucan
other than CD

FIG. 8

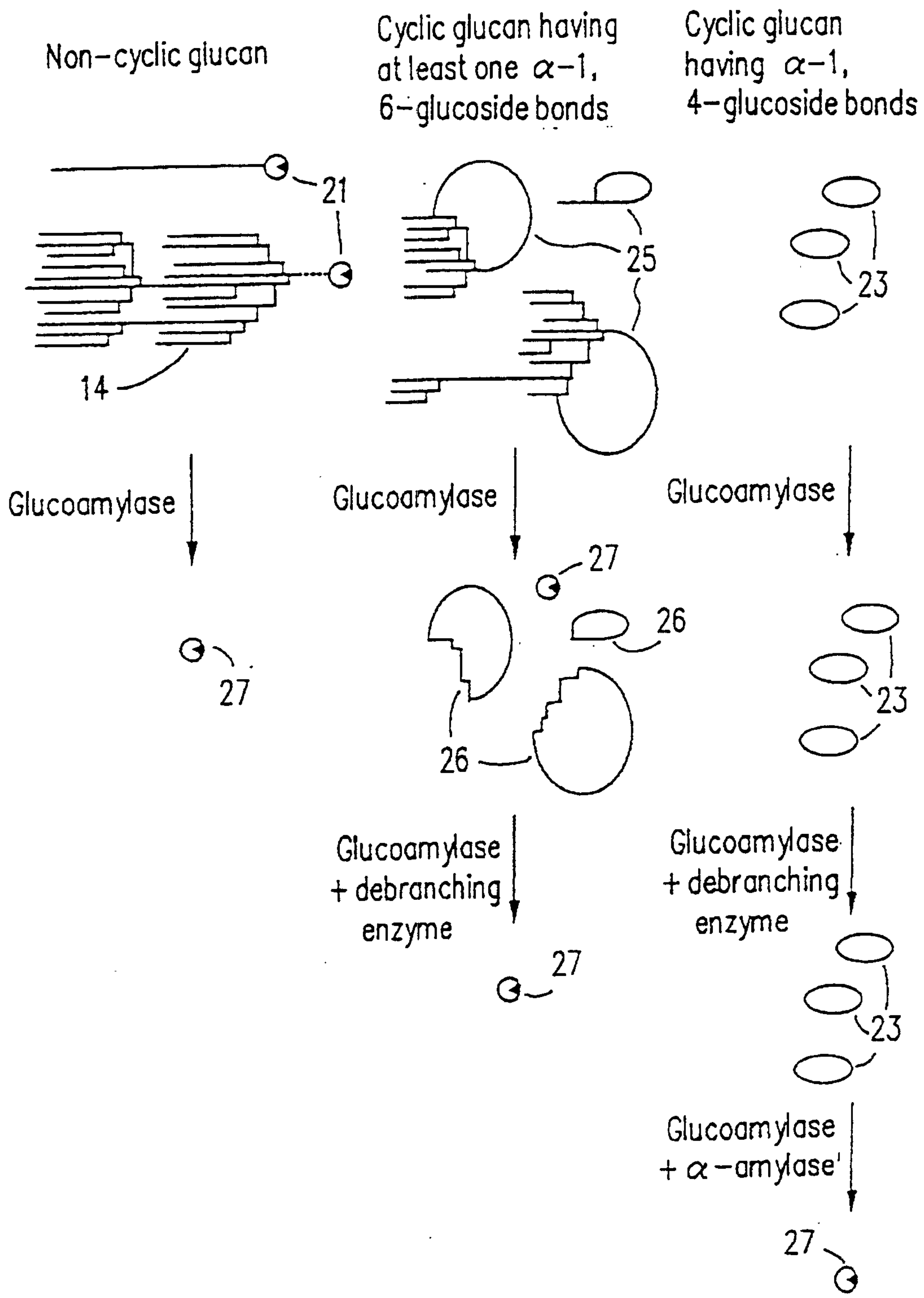

Non-cyclic glucan
Cyclic glucan having at least one α-1, 6-glucoside bonds
Cyclic glucan having α-1, 4-glucoside bonds
21
14
25
23
Glucoamylase
Glucoamylase
Glucoamylase
27
26
26
27
23
Glucoamylase + debranching enzyme
Glucoamylase + debranching enzyme
27
23
Glucoamylase + α-amylase
27

*FIG.9*

| | Conserved region 1 | Conserved region 2 | Conserved region 3 | Conserved region 4 |
|---|---|---|---|---|
| α-amylase | DAVINH | GFRLDAAKH | EVID | FVDNHD |
| Branching enzyme | | | | |
| Derived from E. coli | DWVPGHF | ALRVDAVAS | EFGG | LPLSHDEVVH |
| Derived from Bacillus stearothermophilus 1503R-var. 4 | DWVPGHF | GFRVDAVAN | EFLQ | LPFSHDEVVH |

Primer 1 5'GAYTGGGTNCCNGSNCAYTTY3'

Primer 4 3'WSNGTRCTRCTYCANCANGTR5'

The amino acid sequence with solidline corresponds to a DNA sequence of Primer 1, and the amino acid sequence with wave line corresponds to a DNA sequence of Primer 4.

Y: T or C N: G or A or T or C S: G or C W: A or T R: A or G

*FIG.10*

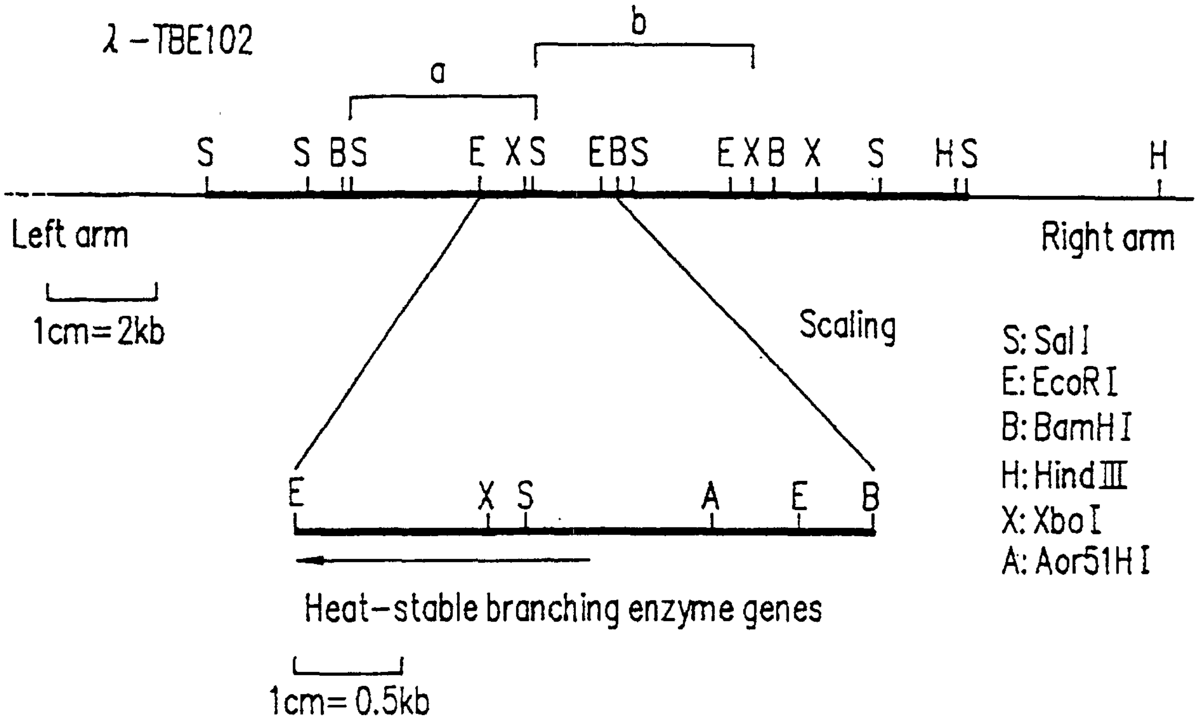
λ-TBE102
a
b
S
S BS
E XS
EBS
EXB X
S
HS
H
Left arm
Right arm
1cm=2kb
Scaling
S: SalI
E: EcoRI
B: BamHI
H: HindIII
X: XboI
A: Aor51HI
E
X S
A
E
B
Heat-stable branching enzyme genes
1cm=0.5kb

*FIG. 11*

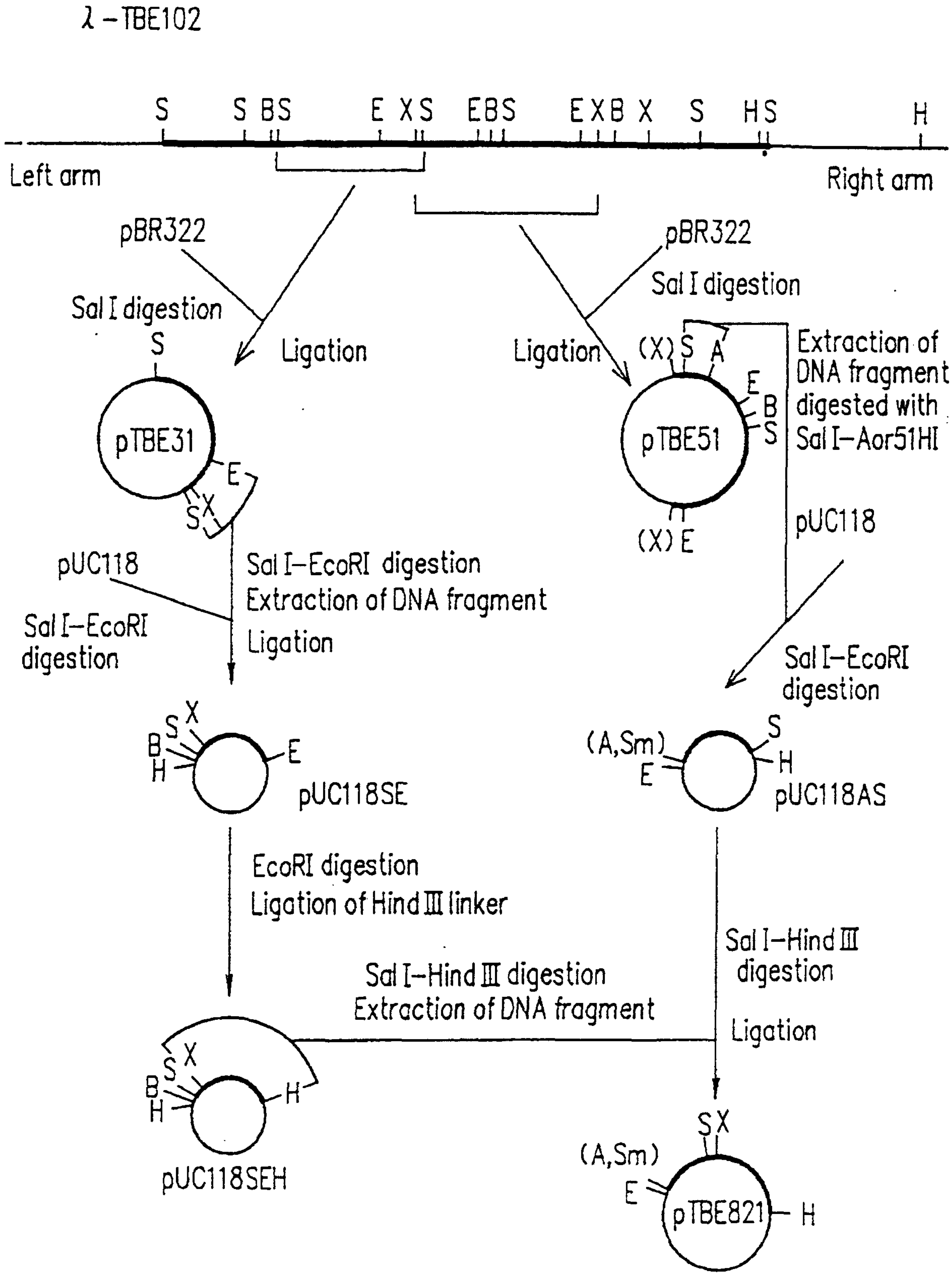
λ-TBE102
S
S BS
E XS
EBS
EXB X
S
HS
H
Left arm
Right arm
pBR322
Sal I digestion
Ligation
pBR322
Sal I digestion
Ligation
S
pTBE31
E
S X
(X) S A
E
B
S
pTBE51
(X) E
Extraction of DNA fragment digested with Sal I-Aor51HI
pUC118
Sal I-EcoRI digestion
Extraction of DNA fragment
Ligation
pUC118
Sal I-EcoRI digestion
pUC118
Sal I-EcoRI digestion
B S X
H
E
pUC118SE
(A,Sm)
E
S
H
pUC118AS
EcoRI digestion
Ligation of Hind III linker
Sal I-Hind III digestion
Sal I-Hind III digestion
Extraction of DNA fragment
Ligation
B S X
H
H
pUC118SEH
S X
(A,Sm)
E
pTBE821
H

FIG.12

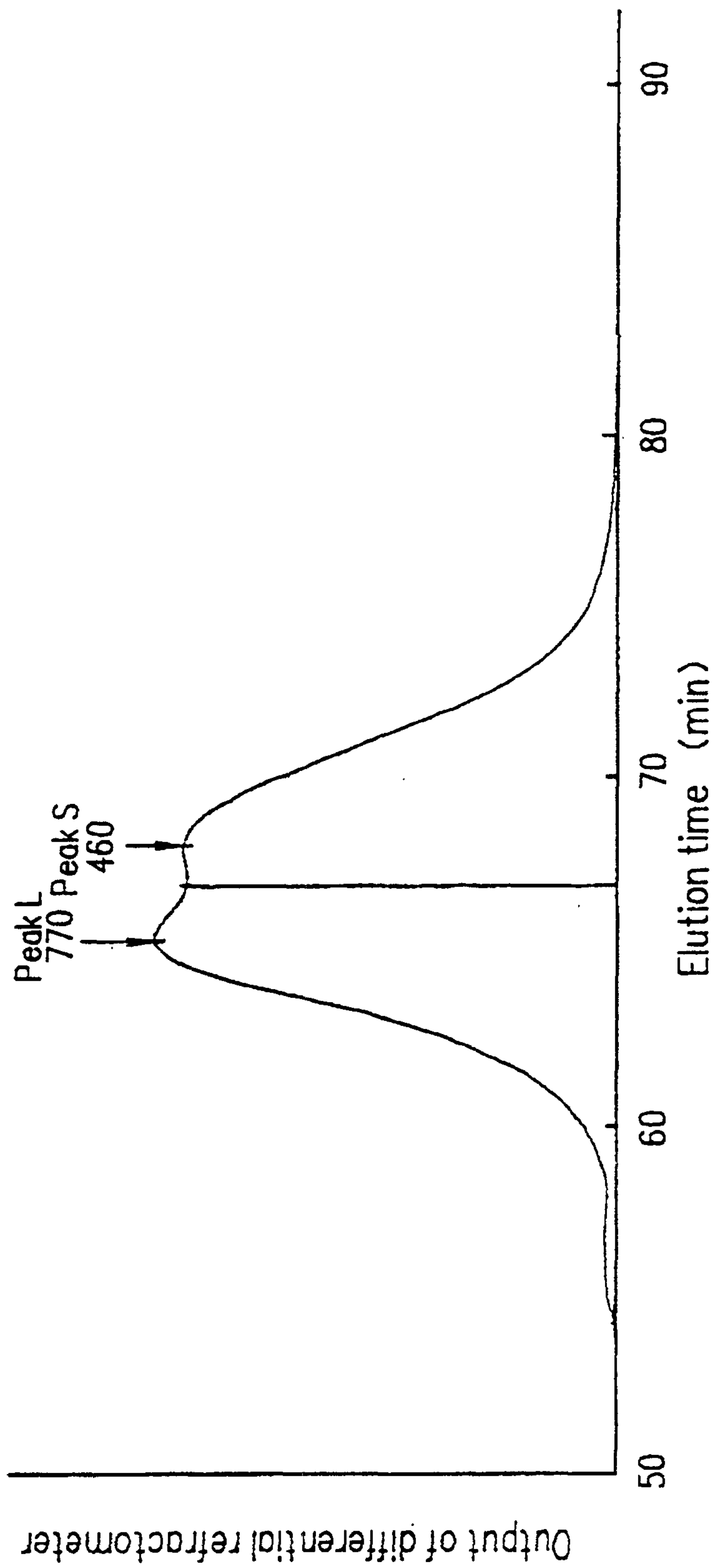
Peak L
770
Peak S
460
Output of differential refractometer
Elution time (min)
50
60
70
80
90

FIG.13

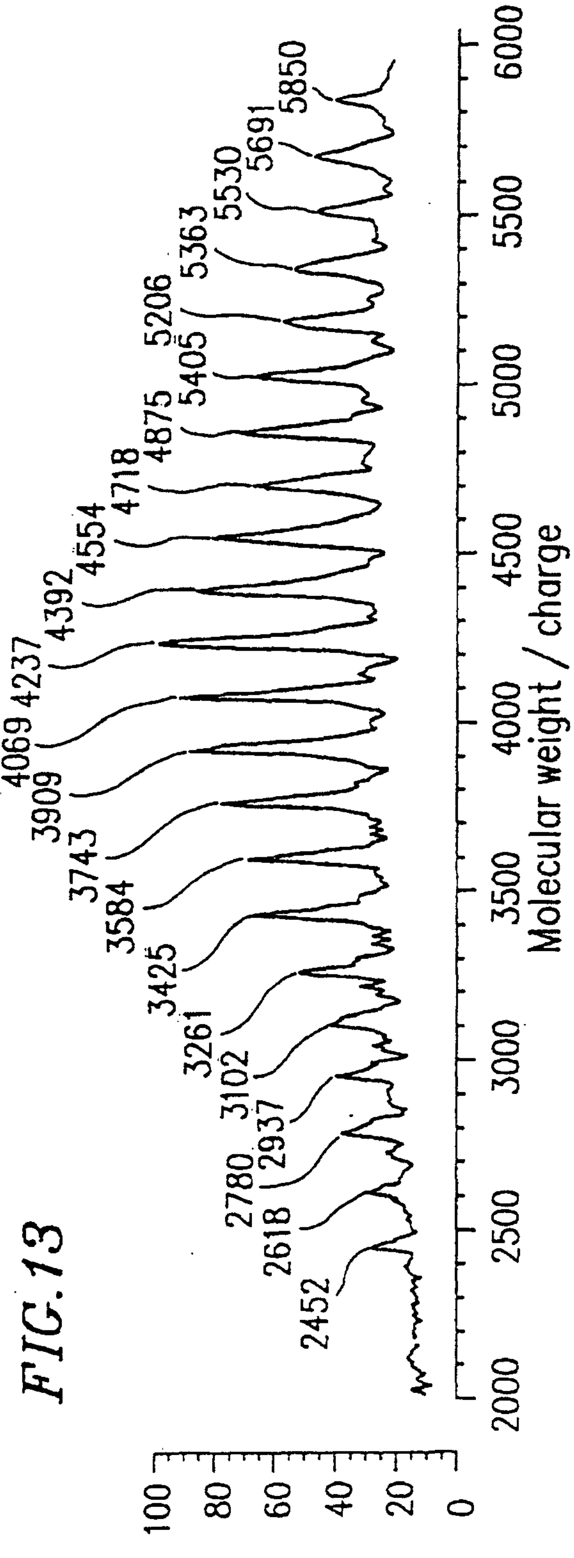
2452
2618
2780
2937
3102
3261
3425
3584
3743
3909
4069
4237
4392
4554
4718
4875
5405
5206
5363
5530
5691
5850
100
80
60
40
20
0
2000
2500
3000
3500
4000
4500
5000
5500
6000
Molecular weight / charge

*FIG.14*

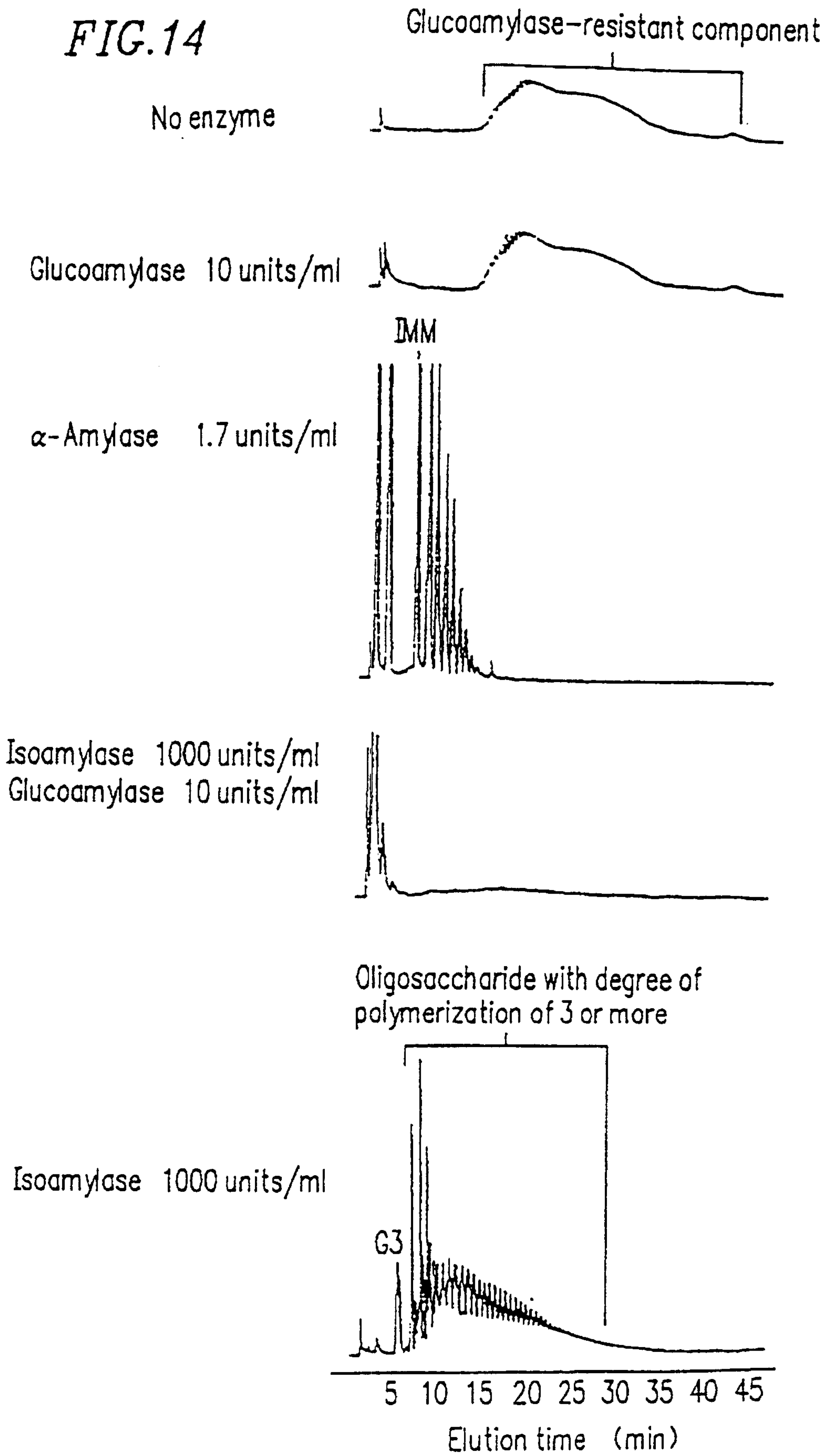
Glucoamylase-resistant component
No enzyme
Glucoamylase 10 units/ml
IMM
α-Amylase 1.7 units/ml
Isoamylase 1000 units/ml
Glucoamylase 10 units/ml
Oligosaccharide with degree of
polymerization of 3 or more
Isoamylase 1000 units/ml
G3
5 10 15 20 25 30 35 40 45
Elution time (min)

*FIG.15*

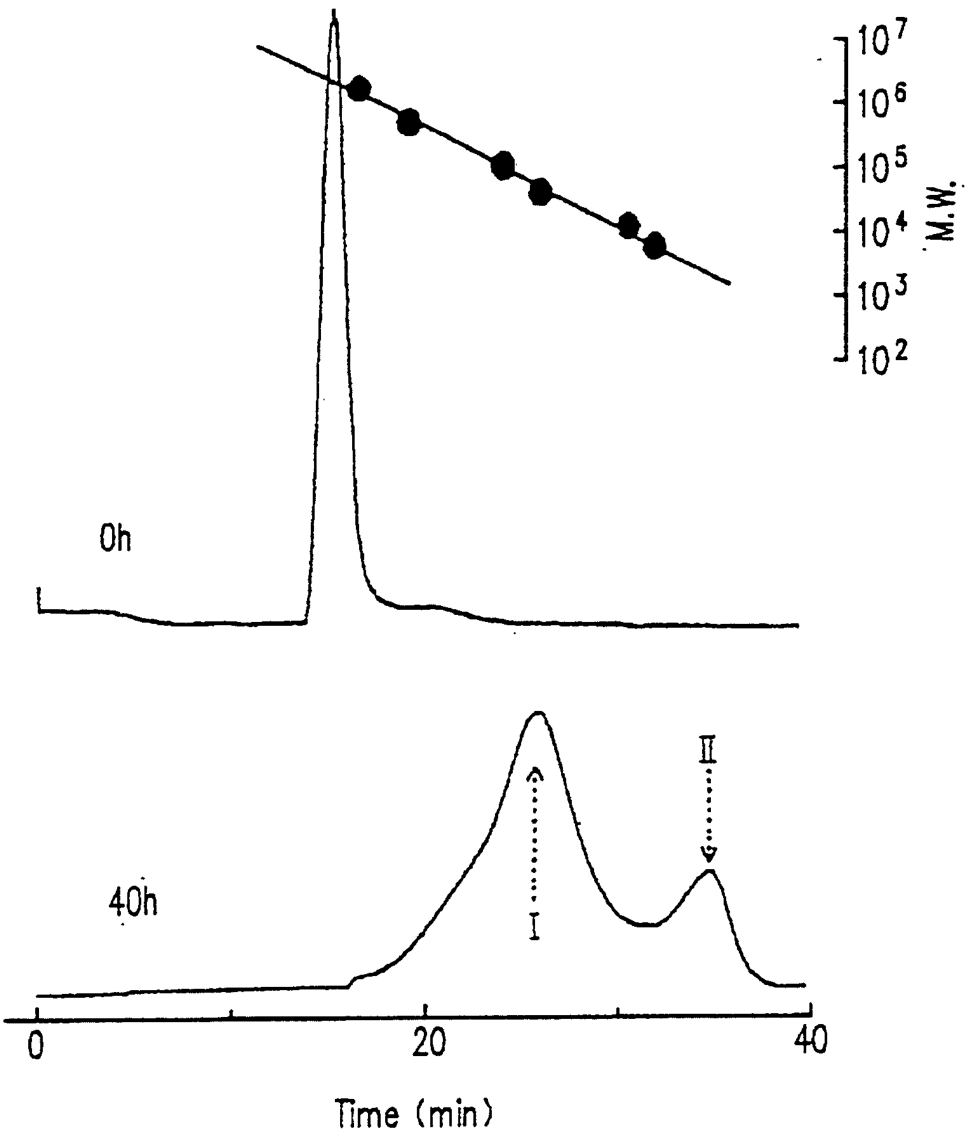

FIG.16

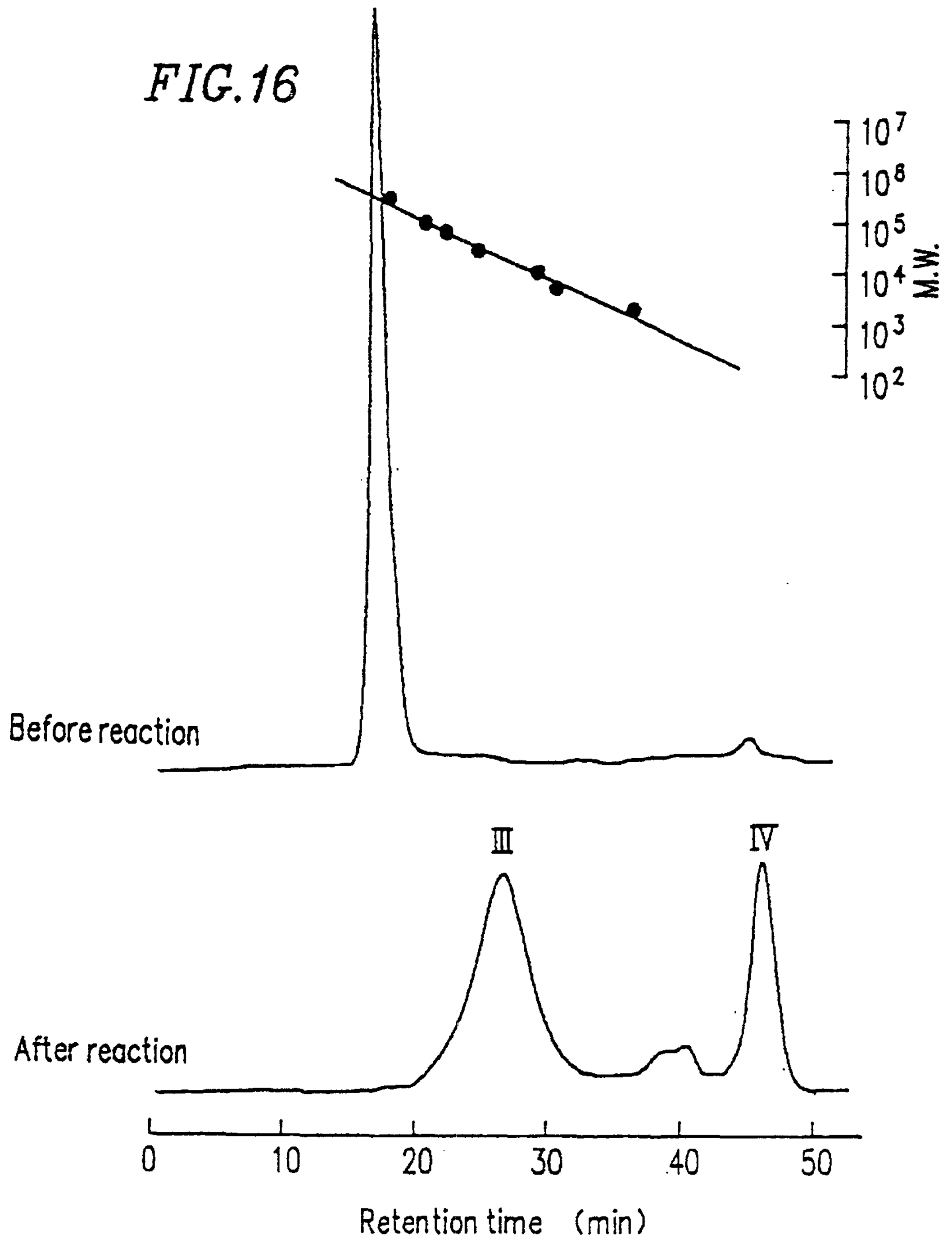
10^7
10^6
10^5
10^4
10^3
10^2
M.W.
Before reaction
III
IV
After reaction
0
10
20
30
40
50
Retention time (min)

*FIG.17*

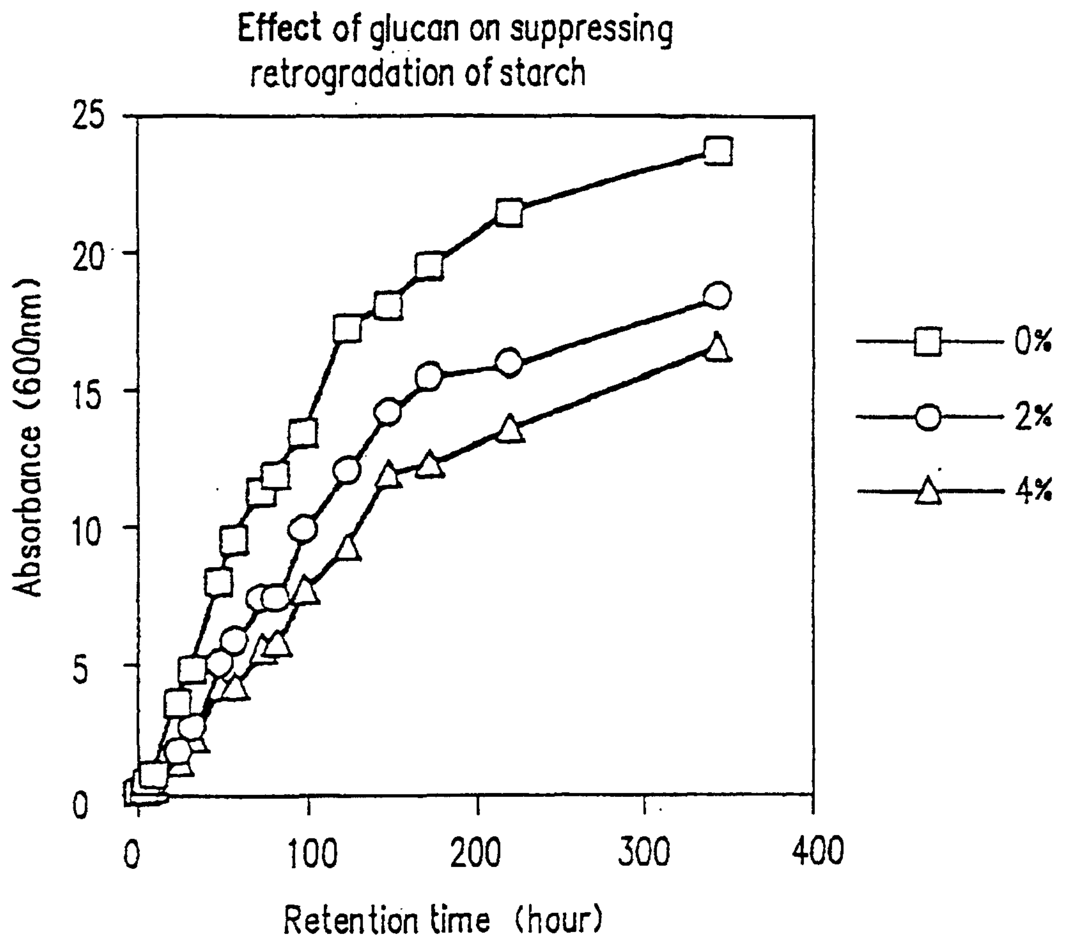
Effect of glucan on suppressing retrogradation of starch
25
20
15
10
5
0
Absorbance (600nm)
0
100
200
300
400
Retention time (hour)
0%
2%
4%